Europäisches Patentamt

European Patent Office

Office européen des brevets

(19)

(11) **EP 1 069 888 B1**

(12) **EUROPEAN PATENT SPECIFICATION**

(45) Date of publication and mention
of the grant of the patent:
**22.09.2004 Bulletin 2004/39**

(51) Int Cl.⁷: **A61K 9/14**, A61B 5/055

(21) Application number: **99915906.4**

(22) Date of filing: **09.04.1999**

(86) International application number:
**PCT/GB1999/001100**

(87) International publication number:
**WO 1999/052505 (21.10.1999 Gazette 1999/42)**

(54) **USE OF PARTICULATE CONTRAST AGENTS IN DIAGNOSTIC IMAGING FOR STUDYING PHYSIOLOGICAL PARAMETERS**

VERWENDUNG VON TEILCHENFÖRMIGEN KONTRASTMITTELN IN DER DIAGNOSTISCHEN BILDERZEUGUNG ZUR UNTERSUCHUNG PHYSIOLOGISCHER PARAMETER

UTILISATION D'AGENTS DE CONTRASTE PARTICULAIRES DANS L'IMAGERIE DIAGNOSTIQUE PERMETTANT D'ETUDIER DES PARAMETRES PHYSIOLOGIQUES

(84) Designated Contracting States:
**AT BE CH CY DE DK ES FI FR GB GR IE IT LI LU MC NL PT SE**

(30) Priority: **09.04.1998 GB 9807840**
**31.12.1998 GB 9828874**

(43) Date of publication of application:
**24.01.2001 Bulletin 2001/04**

(73) Proprietor: **Amersham Health AS**
**0401 Oslo (NO)**

(72) Inventors:
• **FOSSHEIM, Sigrid, Lise**
**N-0401 Oslo (NO)**
• **KLAVENESS, Jo**
**N-1166 Oslo (NO)**
• **BJORNERUD, Atle**
**N-0765 Oslo (NO)**
• **RONGVED, Pal**
**N-1450 Nesoddtangen (NO)**
• **GOLMAN, Klaes Amersham Health R&D AB**
**S-205 12 Malmö (SE)**
• **SKURTVEIT, Roald**
**N-0401 Oslo (NO)**

(74) Representative: **Rollins, Anthony John et al**
**Amersham plc**
**Amersham Place**
**Little Chalfont, Bucks. HP7 9NA (GB)**

(56) References cited:

WO-A-94/28874        WO-A-96/40060
WO-A-97/40858        WO-A-97/48337
US-A- 4 136 683      US-A- 4 914 608
US-A- 5 190 039      US-A- 5 631 141
US-A- 5 720 939

• ALEXANDER A L ET AL: "MICROBUBBLES AS NOVEL PRESSURE-SENSITIVE MR CONTRAST AGENTS" MAGNETIC RESONANCE IN MEDICINE, vol. 35, no. 6, 1 June 1996 (1996-06-01), pages 801-806, XP000594132 ISSN: 0740-3194
• AIME S ET AL: "A NEW YTTERBIUM CHELATE AS CONTRAST AGENT IN CHEMICAL SHIFT IMAGING AND TEMPERATURE SENSITIVE PROBE FOR MR SPECTROSCOPY" MAGNETIC RESONANCE IN MEDICINE, vol. 35, no. 5, 1 May 1996 (1996-05-01), pages 648-651, XP000587869 ISSN: 0740-3194
• YEKTA OEZER A ET AL: "TEMPERATURE- AND PH-SENSITIVE LIPOSOMES" EUROPEAN JOURNAL OF PHARMACEUTICS AND BIOPHARMACEUTICS, vol. 39, no. 3, 1 June 1993 (1993-06-01), pages 97-101, XP000461847 ISSN: 0939-6411
• WILLIAMS G D ET AL: "SIMULTANEOUS DETERMINATION OF INTRACELLULAR MAGNESIUM AND PH FROM THE THREE 31P NMR CHEMICAL SHIFTS OF ATP1" ANALYTICAL BIOCHEMISTRY, vol. 214, 1 January 1993 (1993-01-01), pages 458-467, XP002048884 ISSN: 0003-2697

EP 1 069 888 B1

- **BACIC G ET AL: "OXYGEN TENSION IN A MURINE TUMOR: A COMBINED EPR AND MRI STUDY" MAGNETIC RESONANCE IN MEDICINE,** vol. 30, no. 5, 1 November 1993 (1993-11-01), pages 568-572, XP000404196 ISSN: 0740-3194

**Description**

This invention relates to the use of particulate contrast agents in diagnostic imaging procedures for studying physiological parameters of the subject under investigation.

In diagnostic imaging procedures, e.g. X-ray, MRI, ultrasound, light imaging and nuclear imaging, it has long been known to use contrast agents to facilitate visualization of particular organs or tissues or to identify diseased or malfunctioning regions, ie. generating morphological images.

The present invention is concerned with the use of parenterally administered particulate contrast agents for the quantitative or qualitative study of physiological parameters within the human or non-animal (e.g. mammalian, avian or reptilian, but preferably mammalian) body.

Such parameters include for example pH, temperature, pressure, oxygen tension, carbon dioxide tension, ion tension/concentration the presence or concentration of other body metabolites or enzymes and cell surface properties, e.g. the presence or absence of various cell surface receptors. Parameters such as these may be indicative of the normal or abnormal functioning of the body as a whole or of a particular localized region, e.g. an organ which may or may not be tumorous, infected or otherwise malfunctioning. Likewise variations in such parameters may occur in response to drugs or other treatments administered to the body, e.g. hyperthermic treatment. As a result, quantitative, semi-quantitative or even qualitative determination of such parameters may be used to assess the need for a particular treatment or to monitor the success of a particular treatment.

pH and temperature are particularly important as indicators of abnormality or malfunction.

Several in vivo methods, both imaging techniques and non-imaging techniques, can be used to study physiological parameters, e.g. to diagnose disease. Typical non-imaging techniques include simple blood pressure measurements, electrocardiography or electrocephalography for detection of electric currents in the heart muscle and brain, respectively, and other simple tests performed in doctors' offices or hospitals. Today, a variety of imaging techniques are also used. The most frequently used methods include various X-ray based techniques, MRI, ultrasound and diagnostic methods based on radioactive materials (e.g. scintigraphy, PET and SPECT). Other diagnostic imaging methods include light imaging modalities, Overhauser MR (OMRI), oxygen imaging (OXI) which is based on OMRI, magnetic source imaging (MSI), applied potential tomography (APT) and imaging methods based on microwaves.

The images obtained in X-ray techniques reflect the different densities of structures/organs/tissues in the patient's body. Contrast agents are today used to improve the image contrast in soft tissue examinations. Examples of such contrast agents include gas (negative contrast effect relative to tissue); barium sulphate suspensions; and iodinated agents including ionic monomeric agents, non-ionic monomers, ionic dimers and non-ionic dimers. Typical examples of commercial X-ray contrast agents are Omnipaque® and Visipaque®.

MRI is an imaging method generally based on interactions between radiowaves and body tissue water protons in a magnetic field. The contrast parameter or signal intensity is dependent on several factors including proton density, spin lattice ($T_1$) and spin spin ($T_2$) relaxation times of water protons. Typical commercial MRI contrast agents include Omniscan®, Magnevist® and ProHance®.

Ultrasound is another valuable modality in diagnostic imaging as it does not involve the use of ionizing radiation. In ultrasound examinations the patient is generally exposed to sound waves in the frequency of 1-10 MHz. These sound waves (or ultrasound waves) penetrate through or are reflected from the tissue. The transmitted or reflected sound waves are detected by a "microphone" and form the basis for development of a ultrasound image. Ultrasound imaging is a method of choice in pregnancy checks and birth control and diagnosis of cardiovascular and liver diseases.

Although ultrasound contrast agents have been approved, there is as yet no general use of these agents. The main reason for this is the poor efficacy of the "first generation" agents. The ultrasound contrast agents currently under development are based on encapsulated gas because the reflection of sound from the liquid-gas interface is extremely efficient.

Typical ultrasound contrast agents are gas encapsulated in a sugar matrix, in a shell of denatured albumin/ or partly denatured albumin, in polymers, and in surfactants including phospholipids. A typical ultrasound contrast agent with high contrast efficacy consists of a fluorinated gas bubble (for example $SF_o$ or a perfluorcarbon such as perfluoropropane or perfluorobutane) coated with a mono or multilayer phospholipid membrane. The particle size will generally be around 4 micrometer with very few particles larger than 10 micrometer in diameter. The main indications for such a typical product in the future may be cardiac imaging (cardiac perfusion examinations) and liver imaging.

Nuclear medicine imaging modalities are based upon administration of radioactive isotopes followed by detection of the isotopes, e.g. using gamma camera or positron emission tomography (PET). The most frequently used examination is gamma camera detection of 99-technetium in the form of a chelate, for example a technetium phosphonate chelate for bone scintigraphy.

Light imaging methods are performed using contrast agents that absorb and/or emit light (generally near infrared light).

MSI methods may be performed without contrast agents; however, contrast agents based on magnetic materials improve this technique substantially.

APT based methods can also be performed (like for example thallium scans) without use of contrast agents; again however, contrast agents based on physiologically acceptable ions or other agents with effect on conductivity improve the diagnostic utility of APT.

All these different modalities complement each other with regard to diagnosis based on morphology/anatomy.

However, there has been a great interest in measurement and quantification of various physiological parameters. (See for example J. Magn. Reson. Imaging 1997, 7, 82-90 for a review on physiologic measurements by contrast enhanced MR imaging).

Various methods for measurements of physiologically important parameters have been described in the scientific literature: tissue pH has been measured using near infrared reflectance spectroscopy (J. Clin. Monit. 1996, 12, 387-95); intratumor pH has been measured using $^{19}$F magnetic resonance spectroscopy (Invest. Radiol. 1996, 31, 680-9); 6-fluoropyridoxal polymer conjugates have been suggested as $^{19}$F pH indicators for magnetic resonance spectroscopy (Bioconjug. Chem. 1996, 7, 536-40); spectral imaging microscopy has been used for simultaneous measurements of intracellular pH and $Ca^{2+}$ in insulin-secreting cells (Am. J. Physiol. 1996, 270, 1438-46); fluorescence ratio imaging has been used for measurement of interstitial pH in solid tumors (Br. J. Cancer 1996, 74, 1206-15); a fluorinated pH probe for $^{19}$F magnetic resonance spectroscopy has been used for in vivo pH measurement after hyperthermic treatment of tumors in mice (Acta Radiol. 1996, 3, 5363-4); P-NMR has been used for analysis of intracellular free magnesium and pH in erythrocytes (J. Soc. Gynecol. Investig. 1996, 3, 66-70); intracellular pH has been estimated in developing rodent embryos using computer imaging techniques (Teratology, 1995, 52, 160-8); biscarboxyethyl carboxyfluorescein has been evaluated as in vivo fluorescent pH indicator (J. Photochem. Photobiol. B. 1995, 227, 302-8); the effect of blood flow modification on intra- and extracellular pH has been measured by $^{31}$P magnetic resonance spectroscopy in murine tumors (Br. J. Cancer, 1995, 72, 905-11); intracellular $Ca^{2+}$, pH and mitochondrial function in cultures of rabbit corneal tissue have been studied by digitized fluorescence imaging (In Vitro Cell Biol. Anim. 1995, 31, 499-507); a dual-emission fluorophore has been evaluated for fluorescence spectroscopy of pH in vivo (J. Photochem. Photobiol. B. 1995, 28, 19-23); nuclear magnetic resonance spectroscopy has been used to study lactate efflux and intracellular pH during hypoxia in rat cerebral cortex (Neurosci. Lett. 1994, 178, 111-4); $^{31}$P NMR spectroscopy has been used for imaging of phosphoenergetic state and intracellular pH in human calf muscles after exercise (Magn. Reson. Imaging 1994, 12, 1121-6); multinuclear NMR spectroscopy has been used for studies of regulation of intracellular pH in neuronal and glial tumour cells (NMR Biomed. 1994, 7, 157-166), 5,6-carboxyfluorescein has been used as a pH sensitive fluorescent probe for in vivo pH measurement (Photochem. Photobiol. 1994, 60, 274-9); a fluorinated pH-probe has been used for non-invasive in vivo pH measurements (Invest. Radiol. 1994, 29, 220-2); fluorescence ratio imaging microscopy has been used for non-invasive measurement of interstitial pH profiles in normal and neoplastic tissue (Cancer Res. 1994, 54, 5670-4); 6-fluoro-pyridoxol has been used as probe of cellular pH using F NMR spectroscopy (FEBS Lett. 1994, 349, 234-8); lactate and pH have been mapped in calf muscles of rats during ischemia/reperfusion assessed by in vivo proton and phosphorus magnetic resonance chemical shift imaging (Invest. Radiol. 1994, 29, 217-23); nuclear magnetic resonance spectroscopy has been used for measurement of in vivo and ex vivo pH (Eur. J. Lab. Med. 1996, 4, 143-156); seminaphthofluoresceincalcein has been tested as fluorescent probe for determination of intracellular pH by simultaneous dual-emission imaging laser scanning confocal microscopy (J. Cell Physiol. 1995, 164, 9-16); ampholytic dyes have been proposed for spectroscopic determination of pH in electrofocusing (J. Chromatogr. A 1995, 695, 113-122); EPR spectroscopy has been used for direct and continuous determination of pH values in nontransparent water-in-oil systems (Eur. J. Pharm. Sci. 1995, 3, 21-6); intracellular $Ca^{2+}$ and pH have been imaged simultaneously in glomerular epithelial cells (Am. J. Physiol. Cell Physiol. 1993, 46, 216-230); fluorinated macromolecular probes have been evaluated for non-invasive assessment of pH by magnetic resonance spectroscopy (Bioorg. Med. Chem. Lett. 1993, 2, 187-192); pH has been mapped in living tissue by application of in vivo $^{31}$P NMR chemical shift imaging (Magn. Res. Med. 1993, 29, 249-251); fluorescence spectroscopy has been used to measure temperature dependent aggregation of pH-sensitive phosphatidyl ethanolamine oleic acid-cholesterol liposomes (Anal. Biochem. 1992, 207, 109-113); $^{13}$C NMR spectroscopy has been used to determine intracellular pH (Am. J. Physiol. Cell. Physiol. 1993, 264, C755-C760); $^{31}$P NMR chemical shift imaging has been used for pH mapping of living tissue (Magn. Reson. Med. 1993, 29, 249-251); fluorescent probe and $^{31}$P NMR spectroscopy have been compared for measurement of the intracellular pH of propionibacterium acnes (Can. J. Microbiol. 1993, 39, 180-6); panoramic imaging of brain pH and CBF has been performed during penicillin and metrazole induced status epilepticus (Epilepsy Res. 1992, 13, 49-58); nuclear magnetic resonance spectroscopy has been used to study energy metabolism, intracellular pH and free Mg concentration in the brain of transgenic mice (J. Neurochem. 1992, 58, 831-6); the pH dependence of 5-fluorouracil uptake has been observed by in vivo $^{31}$P and $^{19}$F nuclear magnetic spectroscopy (Cancer Res. 1991, 51, 5770-3); $^{31}$P magnetic resonance spectroscopy has been used to study tumor pH and response to chemotherapy in non-Hodkin's lymphoma (Br. J. Radiol. 1991, 64, 923-8); $^{31}$P magnetic resonance spectroscopy and microelectrodes have been used to evaluate dose-dependent thermal response of tumor pH and energy metabolism (Radiat. Res. 1991, 127, 177-183); hepatic intracellular pH has been studied in vivo by $^{19}$F NMR spectroscopy (Magn. Reson. Med.

1991, 19, 386-392); the relationship between vertebral intraosseous pressure, pH, $pO_2$, $pCO_2$ and magnetic imaging signal inhomogeneity has been evaluated in a patient with back pain (Spine 1991, 16, 239-242); the effect of hypoxia on phosphorus metabolites and intracellular pH in the fetal rat brain have been studied by $^{31}$P NMR spectroscopy (J. Physiol. 1990, 430, 98P); brain pH in head injury has been evaluated using image-guided $^{31}$P magnetic resonance spectroscopy (Ann. Neurol. 1990, 28, 661-7); Se-labeled tertiary amines have been prepared and evaluated as brain pH imaging agents (Nucl. Med. Biol. Int. J. Radiat. Appl. Instrum. Part B 1990, 17, 601-7); $^1$H, $^{31}$P and $^{13}$C nuclear magnetic resonance spectroscopy have been used to study cerebral energy metabolism and intracellular pH during severe hypoxia and recovery in the guinea pig cerebral cortex in vitro (J. Radiat. Appl. Instrum. Part B 1990, 26, 356-369); development of a pH-sensitive contrast agent for H NMR imaging has been reported (Magn. Reson. Med 1987, 5, 302-5); and there have been other references to $^{31}$P NMR studies of pH, see for example Biomed. Res. (Japan) 1989 10, Suppl. 3, 587-597, J. Cereb. Blood Flow Metab. 1990, 10, 221-6, Br. J. Radiol. 1990, 63, 120-4, Pediatr. Res. 1989, 25, 440-4, Radiology 1989, 170, 873-8, Cereb. Blood Flow Metab. 1988, 8, 816-821, J Neuro. Chem. 1988, U51U, 1501-9 abd Am. Heart J. 1988, 116 701-8. WO98/41241 of Nihon Schering discusses MRI techniques which utilise polymers in the monitoring of pH.

One important physiological parameter of great medical interest has been temperature; temperature has been measured by electron paramagnetic resonance spectroscopy (J. Biomech. Eng. 1996, 118, 193-200), an ytterbium chelate has been used as a temperature sensitive probe for MR spectroscopy (Magn. Res. Med. 1996, 35, 648-651), fast imaging techniques have been evaluated in MRI for temperature imaging (J. Magn. Reson. B, 1996, 112, 86-90), $^{31}$P and $^1$H magnetic resonance spectroscopy has been used to study relationship between brain temperature and energy utilization rate in vivo (Pediatr. Res. 1995, 38, 919-925), local brain temperature has been estimated in vivo by $^1$H NMR spectroscopy (J. Neurochem. 1995, 38,1995, 1224-30), magnetic resonance has been used to follow temperature changes during interstitial microwave heating (Med. Phys. 1997, 24, 269-277), the temperature dependence of canine brain tissue diffusion coefficient has been measured in vivo using magnetic resonance echoplanar imaging (Int. J. Hyperthermia 1995, 11, 73-86), temperature dependent ultrasound colour flow Doppler imaging has been carried out of experimental tumours in rabbits (Ultrasound Med. Biol. 1993, 19, 221-9), electrical impedance tomography has been proposed for temperature measurement (Trans ASME J. Biochem. Eng. 1996, 118, 193-200), temperature measurement has been carried out in vivo using a temperature-sensitive lanthanide complex and H magnetic resonance spectroscopy (Magn. Res. Med. 1996, 35, 364-9), body temperature imaging by impedance CT has been carried out (Med. Imag. Tech. (Japan) 1995, 13, 696-702), temperature imaging has been carried out inside the human body using microwaves (Med. Imag. Techn. (Japan) 1995, 13, 691-5), in vivo oxygen tension and temperature have been determined simultaneously using $^{19}$F NMR spectroscopy of perfluorocarbon (Mag. Res. Med. 1993, 29, 296-302), measurement of in vivo pH in normal and tumor tissue has been carried out by localized spectroscopy using a fluorescent marker (Optical Eng. 1993, 32, 239-43), microwave temperature imaging has been proposed (IEEE Trans. Med. Imag. (USA) 1992, 4, 457-69), non-invasive temperature mapping during hyperthermia has been carried out by MR imaging of molecular diffusion (Proceedings of the Annual International Conference of the IEEE 1988, 342-343). There have been other reports of non-invasive and minimally invasive methods for the early detection of disease states by MRI, positron emission tomography, EEG imaging, MEG imaging, SPECT, electrical impedance tomography (APT), ECG imaging and optical diffusion tomography, see for example Proceedings of the SPIE-The International Society for Optical Engineering (USA) 1887 (1993).

The following, predominantly MRI based, techniques have also been reported in the measurement of temperature and temperature changes; Med. Phys 1997, 24(2), 269-277, Int. J. Hyperthermia 1995, 11(5), 409-424, Int. J. Hyperthermia 1992, 8(2), 253-262, Int. J. Hyperthermia (1994), 10(3), 389-394, Radiologe 1998, 38, 200-209, Med Phys 1997, 24(12), 1899-1906, JMRI 1998, 8, 128-135, JMRI, 1998, 8, 160-164, JMRI 1998, 8, 165-174, MRM 1995, 34, 359-367, MRM 1995, 33, 729-731, MRM 1995, 33, 74-81, Radiology 1998, 208, 789-794, JMRI 1996, 7, 226-229, JMRI 1997, 8 188-196, JMRI 1998, 8, 197-202, JMRI 1998, 8, 31-39, JMRI 1998, 8, 121-127, JMRI 1998, 8, 493-502, Int. J. Radiation Oncology Biol. Phys. 1998, 40(4), 815-822, Int. J. Hyperthermia 1998, 14(5), 479-493, Radiology 1995, 196, 725-733, Advances in Radiation Therapy 1998 Eds. Mittal, Purdy and Ang, Kluver Academic Publishers, Chapter 10, pp. 213-245.

Several patents and patent applications which relate to physiological imaging have been published: use of macrocyclic metal complexes as temperature probes for the determination of body temperature using spectroscopic methods with reduced background signals (WO94/27977); new fluorine containing macrocyclic metal complexes from tetraazadodecane derivatives useful for measuring tissue temperature from NMR chemical shift values, and as contrast agents for X-ray or NMR diagnosis (WO94/27978); determining and imaging of temperature change in human body using diffusion coefficients obtained by NMR to determine absolute temperature for individual points of body and temperature differences (WO90/02321); thermographic imaging using a temperature dependent paramagnetic material in an ESR enhanced magnetic resonance imaging apparatus (WO90/02343); fluorosubstituted benzene derivatives useful as agents for in vivo NMR diagnosis, e.g. for measurement of tissue specific pH temperature, redox potentials, etc. (EP-A-368429); a magnetic resonance pulsed heat system for selectively heating a region of a subject that uses pulsed heat from focussed ultrasound equipment to destroy tumor tissue and MRI to provide fast scan images to monitor tissue and temperature with a diffusion sensitive pulse sequence (US-A-5247935); a magnetic resonance pulsed heat system for selectively heating tissue - surgery is performed using localised heating of tissue guided by and monitored by temperature sensitive magnetic resonance imaging and body tissue is heated using a magnetic resonance imaging system having a source and a probe containing a magnetic imaging coil and heating imaging rf source (US-A-5323778); apparatus for hyperthermia treatment of cancer comprising a combined hyperthermia and MRI probe to simultaneously heat a malignant area and monitor temperature, with a filter to isolate signals (WO91/07132); and a temperature measurement method using tomographic techniques of magnetic resonance imaging to measure the temperature of a region indirectly from an intensity change of magnetic resonance signal (US-A-5207222).

The present invention however is based on the understanding that particulate contrast agents may be produced in which the matrix or membrane material for the particles is responsive to a particular physiological parameter and the response is an increased matrix or membrane permeability or chemical or physical breakdown of the membrane or matrix material. This results in a change in the contrast efficacy of the contrast agent which may be correlated to that physiological parameter.

Thus viewed from one aspect the invention provides a method of imaging of an animate human or non-human animal body, which method comprises: administering parenterally to said body a particulate material comprising a matrix or membrane material and at least one magnetic resonance contrast generating species, which matrix or membrane material is responsive to a pre-selected physiological parameter and the response is an increased matrix or membrane permeability or chemical or physical breakdown of the membrane or matrix material whereby to alter the contrast efficacy of said species in response to a change in the value of said parameter; generating image data of at least part of said body in which said species is present; and generating therefrom a signal indicative of the value or variation of said parameter in said part of said body.

Viewed from a further aspect the invention provides a parenterally administrable contrast medium for imaging of a physiological parameter, said medium comprising a particulate material comprising a matrix or membrane material and at least one magnetic resonance contrast generating species, said matrix or membrane material being responsive to said physiological parameter to cause the contrast efficacy of said contrast generating species to vary in response to said parameter and the response is an increased matrix or membrane permeability or chemical or physical breakdown of the membrane or matrix material. In a particularly preferred embodiment, the matrix or membrane material comprises a lipid or lipid mixture having a Tc value between 35 and 80°C, preferably between 37 and 45°C, more preferably between 38 and 43°C (Tc is defined as the gel-to-liquid crystalline phase temperature). In a further preferred embodiment, the matrix or membrane material comprises peptides or one or more polymers.

Viewed from a still further aspect the invention provides the use of a contrast generating species for the manufacture of a particulate contrast medium for use in a method of diagnosis comprising generating a signal indicative of the value of said physiological parameter, the particles of said contrast medium comprising a matrix or membrane material and at least one magnetic resonance contrast generating species, said matrix or membrane material being responsive to said physiological parameter to cause the contrast efficacy of said contrast generating species to vary in response to said parameter and the response is an increased matrix or membrane permeability or chemical or physical breakdown of the membrane or matrix material.

In the method of the invention, the image data generated may if desired be presented as a two or more dimensional spatial image, alternatively they may be presented as a temporal image, again in two or more dimensions. However in the extreme the data may simply provide one or more image values (e.g. numerical values) which either directly or indirectly may be used to provide quantitative or qualitative information (a signal) indicative of the value of the parameter under study. The image data may if desired be presented in visualizable form but alternatively they may simply be a set of data points which are collected and operated on to produce the signal without a visible image actually being generated. The signal indicative of the value of the parameter under study may likewise be generated in the form of a visible image, e.g. a map of the parameter value within the body, or a chart showing variation of the parameter value with time, or it may simply be a calculated numerical value for the parameter or an indication that the parameter is below or above a particular threshold value. Desirably, however, the signal provides a quantitative or at least semi-quantitative value for the parameter, e.g. either in a region of interest or in.a plurality of regions of interest in the body, for example providing a spatial and/or temporal map of the parameter within at least a portion of the body.

Data relating to a physiological parameter may not necessarily also contain information relating to the anatomy of the animal body and thus, a further aspect of the invention relates to the combination of traditional anatomical imaging with physiological imaging to obtain two images, one containing information about a physiological parameter and the other containing anatomical information. The two images may be combined to give one image with both anatomical and physiological information.

Thus, according to a further aspect is provided a method of imaging of an animate human or non-human animal body, which method comprises:

administering parenterally to said body at least one contrast medium comprising a particulate material comprising a matrix or membrane material and at least one magnetic resonance contrast generating species, said matrix or membrane material being responsive to a pre-selected physiological parameter and the response is an increased matrix or membrane permeability or chemical or physical breakdown of the membrane or matrix material, the contrast efficacy thereof is responsive to a change in value of said pre-selected physiological parameter;
generating image data of at least part of said body in which said contrast medium is present; and
generating therefrom a signal indicative of the
value or variation of said parameter in said part of said body and also generating an anatomical image of the same part of the animal body.

The additional use of anatomical information may aid interpretation of the physiological data. An image generated in response to a physiological parameter, a 'physiological image', may be formed using any of the imaging methods and or contrast media described herein. This physiological image can be combined with a conventional image obtained with or without a contrast agent. Suitable contrast agents for use with traditional anatomical imaging are well known in the art for all types of imaging techniques, MRI, X-ray, ultrasound, light and nuclear imaging etc. and many suitable contrast agents for anatomical imaging are discussed herein.

The imaging technique used to obtain physiological data may be the same or different to the imaging technique used to obtain the anatomical image. In a preferred embodiment the imaging technique will be the same, MRI being particularly suitable.

Two separate contrast agents may be used, one for physiological imaging and one for traditional imaging. The two agents can be injected sequentially and the body scanned sequentially with respect to the appropriate imaging techniques and optionally the two images which are generated are then combined. In an alternative embodiment, a single multi-functional contrast agent may be used which is capable of providing both physiological and anatomical information. A multi-functional MRI contrast agent may be used, wherein one of its functions responds to a physiological parameter while a second function provides anatomical information. Although a single contrast agent is applied, the body may be scanned twice and the resulting two images combined.

In a further alternative embodiment a multi-functional contrast agent may be used wherein the components of the agent function as contrast agents for different imaging techniques. Thus, the contrast agent may contain microbubbles to provide contrast in ultrasound imaging and paramagnetic complexes for MRI, being responsive to a physiological parameter according to the invention. Again, the images obtained by scanning according to the two imaging techniques may be combined.

By way of a further example, MRI with hyperpolarised substances will tend to provide good physiological information relating to e.g. pH, temperature or pressure but little or no anatomical information. Thus, the hyperpolarised MR image is advantageously combined with an anatomical image, e.g. by superimposing the images. The two images may be generated separately or at the same time.

The combination of physiological and anatomical imaging may be used to investigate all parts of the human or non-human animal body and any of the physiological parameters discussed herein, particularly pH and temperature. Where the physiological parameter is temperature, changes in the value of the parameter, i.e. temperature changes, may be caused by intrinsic or extrinsic means. Intrinsic means will include cancer, cardiovascular disease and inflammation while extrinsic means include hyperthermia (external heating) treatment.

Thus, the physiological contrast agent may be a contrast agent for hyperthermia.

The imaging technique used in the method of the invention is MRI. In magnetic resonance techniques, signal strength or chemical shift or both may typically be studied. The particulate contrast agent used will accordingly be or contain a material capable of having a contrast or signal generating effect in MRI, e.g. a paramagnetic, ferromagnetic, ferrimagnetic or superparamagnetic material or a precursor therefor, or hyperpolarized NMR active (i.e. non zero nuclear spin) nuclei (e.g. noble gas or $^{13}$C nuclei). The physiological parameter studied using the method of the invention may be any physiochemical parameter capable of affecting the matrix or membrane material of the contrast agent in such a way that the response to the physiological parameter is an increased matrix or membrane permeability or chemical or physical breakdown of the membrane or matrix material, e.g. pressure, temperature, pH, oxygen tension, carbon dioxide tension, enzyme activity, metabolite concentration, tissue electrical activity, tissue water diffusion, ion concentration, particularly $Mg^{2+}$, $Ca^{2+}$ and $Zn^{2+}$, etc. Preferably however it will be selected from blood parameters, e. g. pressure, temperature and pH, in particular in the vasculature rather than the chambers of the heart. Where temperature is being measured, changes may be due to intrinsic factors such as disease or because of external factors, i.e. hyperthermia. It is not envisaged that the parameter be one which does not affect the membrane or matrix, for example flow rate or perfusion density.

A key part of the present invention is that the contrast agent particles should comprise a membrane or matrix material which is responsive to the physiological parameter under investigation so as to alter the contrast efficacy of the contrast agent and the response is an increased matrix or membrane permeability or chemical or physical break-

EP 1 069 888 B1

down of the membrane or matrix material. The manner in which the membrane or matrix responds will depend on the particular combination of imaging modality, physiological parameter and contrast generating material selected. Typically however the response involves a change in membrane or matrix permeability to one or more species (e.g. water or gases) or chemical or physical breakdown of the membrane or matrix material.

Such a response may thus for example involve release from the particulate contrast agent of water-soluble contrast generating moieties that are capable of being taken up into the extracellular fluid outside the vasculature. Particular examples of physiological parameter responsive particulate contrast agents will be described in greater detail below.

Thus one embodiment of the invention relates to thermosensitive paramagnetic particulate compositions for temperature MRI-mapping of the human body.

Another aspect of the present invention is to use one or more of the thermosensitive particulate compositions for temperature mapping in imaging guided hyperthermia treatment.

Another embodiment of the present invention relates to pH sensitive particulate compositions for determination of pH in the body. By way of example the active contrast agent (or indicator or probe) may be a paramagnetic, magnetic or fluorinated compound detectable by MRI.

Yet another embodiment of the invention relates to particulate compositions as contrast agents or as in vivo indicators or probes for detection of oxygen concentration/tension in the tissue using modalities such as MRI or Overhauser MRI.

Another aspect of the present invention relates to particulate compositions as contrast agents or as in vivo indicators or probes in combination with a targeting ligand, wherein said targeting ligand targets cells or receptors selected from the group consisting of myocardial cells, endothelial cells, epithelial cells, tumor cells, brain cells, and the glycoprotein GPIIb/IIIa receptor, for detection of changes in physiological parameters and/or quantification/ semi-quantification of physiological parameters relevant for diagnosis of disease.

Further examples of targeting ligands which can be used are:

i) Antibodies, which can be used as vectors for a very wide range of targets, and which have advantageous properties such as very high specificity, high affinity (if desired), the possibility of modifying affinity according to need etc. Whether or not antibodies will be bioactive will depend on the specific vector/target combination. Both conventional and genetically engineered antibodies may be employed, the latter permitting engineering of antibodies to particular needs, e.g. as regards affinity and specificity. The use of human antibodies may be preferred to avoid possible immune reactions against the vector molecule.

A further useful class of antibodies comprises so-called bispecific antibodies, i.e. antibodies having specificity for two different target molecules in one antibody molecule. Such antibodies may, for example, be useful in promoting formation of bubble clusters and may also be used for various therapeutic purposes, e.g. for carrying toxic moieties to the target. Various aspects of bispecific antibodies are described by McGuinness, B.T. *et al.* in *Nat. Biotechnol.* (1996) **14**, 1149-1154; by George, A.J. *et al.* in *J. Immunol.* (1994) **152**, 1802-1811; by Bonardi *et al*. in *Cancer Res.* (1993) **53**, 3015-3021; and by French, R.R. *et al*. in *Cancer Res.* (1991) **51**, 2353-2361.

ii) Cell adhesion molecules, their receptors, cytokines, growth factors, peptide hormones and pieces thereof. Such vectors/targeting ligands rely on normal biological protein-protein interactions with target molecule receptors, and so in many cases will generate a biological response on binding with the targets and thus be bioactive; this may be a relatively insignificant concern with vectors which target proteoglycans.

iii) Non-peptide agonists/antagonists or non-bioactive binders of receptors for cell adhesion molecules, cytokines, growth factors and peptide hormones. This category may include non-bioactive vectors which will be neither agonists nor antagonist but which may nonetheless exhibit valuable targeting ability.

iv) Oligonucleotides and modified oligonucleotides which bind DNA or RNA through Watson-Crick or other types of base-pairing. DNA is usually only present in extracelluar space as a consequence of cell damage, so that such oligonucleotides, which will usually be non-bioactive, may be useful in, for example, targeting of necrotic regions, which are associated with many different pathological conditions. Oligonucleotides may also be designed to bind to specific DNA- or RNA-binding proteins, for example transcription factors which are very often highly overexpressed or activated in tumour cells or in activated immune or endothelial cells. Combinatorial libraries may be used to select oligonucleotides which bind specifically to possible target molecules (from proteins to caffeine) and which therefore may be employed as vectors for targeting.

v) DNA-binding drugs may behave similarly to oligonuclotides, but may exhibit biological acitvity and/or toxic effects if taken up by cells.

vi) Various small molecules, including bioactive compounds known to bind to biological receptors of various kinds. Such vectors or their targets may be used to generate non-bioactive compounds binding to the same targets.

vii) Targeting ligands may be selected from combinatorial libraries without necessarily knowing the exact molecular target, by functionally selecting (in vitro, ex vivo or in vivo) for molecules binding to the region/structure to be imaged.

ix) Proteins or peptides which bind to glucosamino-glycan side chains e.g. heparan sulphate, including glucosaminoglycan-binding portions of larger molecules, since binding to such glucosaminoglycans side chains does not result in a biological response. Proteoglycans are not found on red blood cells, thus eliminating undesirable adsorption to these cells.

The particulate contrast agent may thus be used for quantification/semi-quantification of a physiological parameter which is relevant for diagnosis of disease. The particulate contrast agent may be triggered into giving a measurable signal difference either by the target parameter itself (e.g. the local temperature, pH or pressure or by binding to the particular cell surface receptors of interest) or by a chemical or biological response of the target parameter (e. g. release of enzymes or local variation in pH or temperature due to cellular reactions). The particulate agent may thus respond to, identify and/or quantitatively or semi-quantitatively determine bacteria, viruses, antibodies, enzymes, drugs, toxins, etc.

Another aspect of the present invention relates to intravenous particulate compositions as contrast agents or as in vivo indicators or probes with long vascular half life (reduced liver uptake) for detection of changes in physiological parameters and/or quantification/semiquantification of physiological parameters relevant for diagnosis of disease.

The particulate contrast agent used according to the invention may be a solid material, a porous material, a liquid crystal material, a gel, a plastic material, a material having one or more walls or membranes or liquid particles, e.g. emulsion droplets or gas based particles, e.g. micro bubbles. The particles can also be thermodynamically stabilised, e.g. micro emulsion droplets or surfactant micelles. The chemical composition of the particulate material can be one simple chemical compound or a mixture of two or more chemical compounds. Generally it will comprise two or more different chemical entities, at least one of which is a matrix or membrane forming material and at least one other of which is a magnetic resonance contrast generating species. The composition can consist of solid material(s) only or it may be a mixture of different solids/liquids/gases. The particulate will generally have a mean particle size (e.g. as determined by particle size analyzers such as laser light scattering apparatus or Coulter counters) in the range 0.001 to 20 $\mu$m, more preferably 0.01 to 10 $\mu$m, especially 0.05 to 7 $\mu$m. Such particles are often described in the literature as particles, colloids, emulsions, droplets, microcrystals, nanocrystals, microparticles, nanoparticles, vesicles, liposomes, bubbles, microspheres, microbubbles, coated particles, microballons and the like.

The term "polymer" as used herein refers to any chemical compound with more than 10 repeating units. A polymer can be naturally occurring, synthetic, or semisynthetic. Semisynthetic polymers are polymers that are produced by synthetic modification of naturally occurring polymers. Compounds with 2 to 10 repeating units are herein generally referred to as "oligomers" and likewise may be natural, synthetic or semisynthetic.

The term "surface active compound" or "surfactant" is used herein to refer to any chemical compound having at least one hydrophilic functional group and at least one hydrophobic (lipophilic) group. In a multiphase system, surface active compounds will commonly accumulate at the interface.

The term "lipid" is used herein to refer to naturally-occurring compounds, synthetic compounds and semisynthetic compounds which are surface active compounds and have structures similar to fatty acids, waxes, mono-, di- or tri-glycerides, glycolipids, phospholipids, higher ($C_{10}$ or greater) aliphatic alcohols, terpenes and steroids.

The term "gas" is used herein to refer to any compound or a mixture of compounds with sufficiently high vapor pressure to be at least partly in the gas phase at 37°C.

Gaseous contrast generating species may be used in MRI.

Typical examples of gas types that change contrast property as a result of the physiological parameters in the surrounding tissue include: gases that change properties (e.g. lose hyperpolarization or change other magnetic properties) upon contact with body fluids or components, including dissolved components, thereof. Preferred gases include hydrogen, oxygen, nitrogen, noble gases (including hyperpolarized gases), carbon dioxide, fluorinated gases (e.g. sulphur hexafluoride, fluorohydrocarbons, perfluorocarbons and other fluorinated halogenated organic compounds in gas phase), and low molecular weight hydrocarbons. Preferred gases also include any pharmaceutically acceptable gas mixture like for example air and air/perfluorocarbon mixtures. Preferably, the perfluorocarbon gas is selected from perfluoromethane, perfluoroethane, perfluoropropanes and perfluorobutanes. Any physiologically acceptable gas precursor can be used. Among suitable gas precursors are compounds that form a gas as a result of a chemical reaction (for example compounds sensitive to pH, for example carbonic acid, aminomalonic acid or other acceptable pH sensitive gas generating substances). Other suitable gas precursors are compounds that form a gas as a result of other physiological conditions like for example temperature, oxygen, enzymes or other physiological parameters/compounds relevant for body tissue (whether in the normal or diseased state) or which are activated to a gas forming state as a result of an interaction with an external stimulus (e.g. photoactivation, sono-activation etc.).

The encapsulation material can be any material such as for example lipids, phospholipids, surfactants, proteins, oligomers and polymers. Such materials are chosen to respond to the pre-selected physiological parameter in such a way, that the response is an increased matrix or membrane permeability or chemical or physical breakdown of the membrane or matrix material, e.g. to dissolve, melt, collapse, weaken, increase porosity, or otherwise break down, change phase or change size (e.g. by aggregation due to change in surface charge, for example in response to local $Ca^{2+}$ and/or $Mg^{2+}$ concentration) in response to the physiological parameter, e.g. to allow release of the magnetic resonance contrast generating species into the surrounding fluid, or to allow body fluid or components thereof to come into contact with the contrast generating species, or to raise contrast agent species local concentration above the detection limit, etc. In this way the contrast generating effect of the magnetic resonance contrast generating species may be dispersed (e.g. into the extracellular fluid space), switched on or increased (e.g. by generation of a contrast generating species such as a gas or by increasing water contact (for a positive ($T_1$ effect) MR contrast agent such as a gadolinium chelate)), or switched off or decreased (e.g. by destruction of the compartmentalization required for a negative ($T_2$ effect) MR contrast agent such as a dysprosium chelate, or by quenching of a radical or depolarization of a hyperpolarized nucleus or dissolution of a blood soluble gas). Moreover a porous solid matrix, e.g. a zeolite, may be impregnated with the contrast generating species with the pore mouths then being closed off totally or partially using a material which breaks down, melts or dissolves when the relevant physiological parameter (e.g. pH, temperature, enzyme concentration) in the surrounding body fluid is above or below a pre-set value.

The particulate contrast agent used according to the invention may respond to physiological parameters in several different ways. In one aspect, the particulate contrast agent may respond to physiological parameters by accumulation in the area where a certain value for a particular parameter is fulfilled, compared to areas where it is not. In another aspect of the invention, the particulate contrast agent responds by accumulation in areas where the physiological parameter value is not fulfilled. In yet another aspect of the invention, the particulate contrast agent responds to a given parameter by disintegration, the disintegration being dissolution or chemical breakdown. Especially advantageous is a response to a physiological parameter by leakage or other transport means in/out of the particles.

When a particulate composition responds by disintegration or transport, changes in contrast effect may be achieved by exposing otherwise invisible/shielded contrast agents or altering the distribution of contrast agents. Especially advantageous are particulate compositions where the contrast effect is gained by interaction with the environment. In this case, both transport of the contrast agent and transport of the actual environmental component may be utilized for detection of physiological parameters. An example is MRI contrast agents where an increased degree of water access/transport to the contrast agent leads to the measured contrast enhancement. In this case, response to a physiological parameter may be an increased rate of water transport in/out of the particulate.

The leakage or an increased transport rate of solutes in/out of a particulate may be accomplished in a variety of ways. All kinds of phase transitions may be utilized to induce leakage/transport.. For instance, a solid particle/membrane may become leaky when it is melted, the process being sensitive to temperature. Phase transitions involving a gas phase may be used to respond to pressure as a physiological parameter. An especially useful aspect of the present invention is particles comprising liquid crystalline material as for example liposomes, niosomes or other vesicles. Liquid crystalline materials may undergo several different phase changes which may induce leakage and/or increase the transport rate of solutes or even breakdown of the particle. For example, the gel to liquid crystalline phase transition of phospholipids may increase the liposome permeability and increase the transport rate or induce leakage of solutes on heating and hence temperature sensitivity..The lamellar to reversed hexagonal phase transition will also induce leakage since the liposomes require lipids in lamellar, gel or other layered phase structure. The lamellar to reversed hexagonal phase transition may be induced by pH, electrolytes, and changes in the chemical environment such as targeting, enzymes, antibodies etc. The suitable parameter to respond to may be tuned by selection of the membrane composition and processing. Other phase transitions such as lamellar to cubic phases, lamellar to microemulsion phases or lamellar to normal hexagonal phase may also be used to introduce leakage.

Gel based particles or gel-surrounding particles (e.g. particles made by coacervation) may respond to a physiological parameter by, for example, a lowering of the viscosity of the gel. Such viscosity lowering may for example be obtained by temperature, pH or electrolytes such as $Ca^{2+}$ or $Mg^{2+}$ and the particles are thus sensitive to these parameters. Such parameters may also induce phase separation in the gel particles, leading to leakage of liquid and phase separation of the polymer which comprises the gel. These mechanisms may in turn influence a parameter such as water leakage and exposure of, e.g. paramagnetic chelates to water and hence lead to a change in MRI contrast.

Particles or membranes composed of solid polymer may also respond to physiological parameters. For instance temperature may change the glass transition temperature of the polymer, and hence induce phase transitions in the polymer membrane, which in turn may influence a parameter such as water transport which influences the contrast efficacy of the contrast agent.

Particles which at least in part are composed of or stabilised by water soluble polymers e.g. peptides, may respond to physiological parameters by alternation in the peptide conformation. For instance peptides may undergo an $\alpha$ - helix to $\beta$ - sheet transition or vice versa and hence influence a parameter which in turn effects contrast. Also transitions to/from $\alpha$ - helix or $\beta$ - sheet to random coil may influence a parameter such as membrane permeability, particle stability against aggregation/flocculation or even fusion, or particle dissolution or precipitation which in turn alters the contrast efficacy of the magnetic resonance contrast agent.

Leakage may also be controlled by entities forming channels or other transport routes through the membrane of a particle. These channels may control the transport of molecules in/out of the particle, and be quite selective for, e.g., ions. For instance the protein tubulin which forms microtubules in absence of $Ca^{2+}$ may induce a higher leakage

in presence of $Ca^{2+}$ than in absence of $Ca^{2+}$ and hence be $Ca^{2+}$ sensitive. Other proteins/enzymes which may control the transport of substance in/out of a vesicle, include erythrocyte anion transporter, erythrocyte glucose transporter, $Na^+$-$K^+$ ATPase ($Na^+$/$K^+$ pump), $Ca^{2+}$ - ATPase ($Ca^{2+}$ pump) and Bacteriorhodopsin ($H^+$ - pump). Also biosurfactants such as iturins, esperine, bacillomycins, mycosubtilin, surfactin and similar substances may be used as membrane components to induce/prevent leakage by response to external parameters since these molecules may respond by changes in secondary and tertiary structure as well as self-assembly properties on influence from extrinsic parameters.

The contrast generating species in MR contrast agents used according to the invention will generally be a paramagnetic, superparamagnetic, ferrimagnetic or ferromagnetic compound and/or a compound containing other non zero spin nuclei than hydrogen, e.g. $^{19}F$, $^{13}C$, $^{15}N$, $^{29}Si$, $^{31}P$ and certain noble gases, such as $^{129}Xe$ or $^{3}He$. Preferred as paramagnetic compounds are stable free radicals, and compounds (especially chelates) of transition metal or lanthanide metals, e.g. manganese compounds, gadolinium chelates, ytterbium chelates and dysprosium chelates. Preferred magnetic (e.g. superparamagnetic) compounds are $\gamma$-Fe.O., Fe.O and other iron/metal oxides with high magnetic susceptibility. Preferred fluorinated compounds are compounds with relative short $^{19}F$ T-relaxation times. Other preferred fluorinated compounds according to the present invention are fluorinated pH-probes, such as compounds described in EP-0447013 of Schering A.G. and ZK-150471 described by Y. Aoki in Invest. Radiol 1996, 34, 680-689. Examples of MR contrast effective materials are well known from the patent literature, see for example the patent publications of Nycomed, Salutar, Sterling Winthrop, Schering, Squibb, Mallinckrodt, Guerbet and Bracco.

In general, there are two types of contrast generating species useful in MR contrast agents for use according to the invention: species that change contrast property as a result of the physiological parameters in the surrounding tissue; and species that are inert to physiology but change contrast properties as a result of an interaction between coating material/encapsulation material and physiology. Typical examples here will be GdDTPA, GdDTPA-BMA, Gd-DOTA, GdHPDO3A, PrDO3A-derivatives and Tm chelates in thermosensitive liposomes or in pH-sensitive vesicles.

Typical examples of species that change contrast property as a result of the physiological parameters in the surrounding tissue include: paramagnetic chelates that change relaxation properties and/or change chemical shift as a result of temperature, paramagnetic chelates that change coordination number and thereby relaxation properties and/or shift properties as a function of pH, paramagnetic compounds, for example manganese compounds (Mn(2+)/Mn(3+)), europium compounds (Eu(2+), Eu(3+)) and free radicals (radical, no radical) that change relaxation properties and/or shift properties as a result of oxygen tension/concentration or as a result of redox potential in the surrounding tissue, paramagnetic and magnetic compounds that change relaxation/shift properties as a result of enzymatic activity (for example with enzymatic cleavage of paramagnetic chelates from macromolecules conjugated thereto causing a change in correlation time and/or water coordination) and paramagnetic chelates that change properties as a result of concentration of ions in the tissue, e.g. due to changes in water coordination.

Paramagnetic compounds have, according to the present invention, either an effect on the relaxation times ($T_1$ or $T_2$) or an effect on chemical shift. Typical compounds that change relaxation times are gadolinium chelates, manganese compounds and superparamagnetic iron oxides. Europium chelates, on the other hand, are well-known chemical shift compounds. The effect on chemical shift is related to temperature. Based on this, macrocyclic paramagnetic chelates like 2-methoxyethyl substituted PrDO3A and 1,4,7,10-tetraazacyclododecane-1,4,7,10-tetrakis(methylene phosphonate thulium complex) have been suggested as temperature probes (see WO 94/27977 (Platzek, Schering)) and C.S. Zuo et al. in J. Magn. Res. 133 53-60 (1998)). All these paramagnetic compounds can be used according to the present invention. In vivo temperature measurements have been of great interest because temperature is an important physiological parameter related to several indications including cancer, cardiovascular diseases and inflammation. Local monitoring of temperature will also be of great value during hyperthermia treatment.

Magnetic resonance contrast generating species can be released from the matrix/encapsulation material as a result of increased temperature and thereby change their contrast property or distribute to other tissues than the particulate product. Alternatively for an MR active temperature sensitive agent, a change in contrast efficacy may occur due to an increased permeability of the matrix/encapsulation material, and, hence, to an increased rate of water transport across the matrix/encapsulation material, even if the agent itself does not leave the matrix/encapsulation material.

Typical examples of temperature sensitive particulate materials are temperature sensitive liposomes, these being suitable for use with MRI. These liposomes take advantage of the fact that the membrane permeability is markedly increased at the gel-to-liquid crystal phase transition temperature ($T_c$) of their membrane lipids. Also, possibly depending upon the membrane properties and the nature of the MR active agent, leakage of the agent may occur. Liposomes made from specific phospholipids or a specific blend of phospholipids may be stable up to 37°C but exhibit an increased water permeability or/and leak as they pass through an area of the body in which the temperature is raised, e.g. to 40 to 45°C, as a result of a disease process or an external heating. Table 1 below shows the transition temperature of various saturated phosphatidylcholines.

Table 1

| Phosphatidylcholines (PC) | Transition temperature Tc (°C) |
|---|---|
| 12:0 | -1 |
| 13:0 | 14 |
| 14:0 | 23 |
| 15:0 | 33 |
| 16:0 | 41 |
| 17:0 | 48 |
| 18:0 | 55 |
| 19:0 | 60 |
| 20:0 | 66 |
| 21:0 | 72 |
| 22:0 | 75 |
| 23:0 | 79 |
| 24:0 | 80 |

Table 2 below shows the phase transition of various unsaturated phosphatidylcholines.

Table 2

| Phosphatidylcholines (PC) | Transition temperature Tc (°C) |
|---|---|
| 12:1 | -36 |
| 18:1c9 | -20 |
| 18:1t9 | 12 |
| 18:1c6 | 1 |
| 18:2 | -53 |
| 18:3 | 60 |
| 18:4 | -70 |

Table 3 below shows the phase transition temperature of various asymmetric phosphatidylcholines.

Table 3

| Phosphatidylcholines (PC) | Transition temperature Tc (°C) |
|---|---|
| 14:0-16:0 | 35 |
| 14:0-18:0 | 40 |
| 16:0-14:0 | 27 |
| 16:0-18:0 | 49 |
| 16:0-18:1 | -2 |
| 16:0-22:6 | -27 |
| 16:0-14:0 | 30 |
| 18:0-16:0 | 44 |
| 18:0-18:1 | 6 |
| 18:1-16:0 | -9 |
| 18:1-18:0 | 9 |

Table 4 below shows the phase transition temperature for various saturated symmetric phosphatidylglycerols (PG) in the form of their sodium salts.

Table 4

| Phosphatidylglycerols (PG) | Transition temperature Tc (°C) |
|---|---|
| 12:0 | -3 |

Table 4   (continued)

| Phosphatidylglycerols (PG) | Transition temperature Tc (°C) |
|---|---|
| 14:0 | 23 |
| 16:0 | 41 |
| 18:0 | 55 |

Tables 1-4 are based on information from the product catalogue of Avanti Polar Lipid Inc., USA.

Accordingly, phospholipids or blends of phospholipids may be selected to give products with the correct Tc for thermosensitive liposomes for diagnostic use. Typical blends for preparation of thermosensitive liposomes for diagnostic use are mixtures of dipalmitoylphosphatidylcholine (DPPC) and dipalmitoylphosphatidyl glycerol (DPPG) and distearylphosphatidylcholine (DSPC).

Particulate contrast agents may also respond to temperature by utilizing the conformational temperature sensitivity of certain polymer systems. An example is poly(N-isopropyl acrylamide) which phase separates at 37°C. Hence particles comprising contrast agents will become leaky dependent on temperature (see Hoffmann et al. Macromol. Symp. 118: 553-563 (1997)).

Other examples of temperature sensitive matrices/coatings are lipid suspensions/emulsions containing the contrast generating species or other particulate or particulate like formulations that release the magnetic resonance contrast generating species or change properties as a result of changes in temperature.

If the parameter under study is capable of manipulation, e.g. by treatment with drugs, external application of heat etc., it may be used to study the efficacy of such treatment or localized such treatment may be used to cause a change in contrast efficacy which in turn may be used to measure parameters such as organ perfusion. Thus for example external application of heat at, near or upstream of an organ of interest may be used to cause release from the particles of a contrast agent which may diffuse into the organ and so to detect blood perfusion (or lack of perfusion) in that organ. In this context one might administer a thermally sensitive particulate agent in connection with an external heating to follow the heat transport in parts of the body. Heat transport *in vivo* is directly connected to blood flow through the bioheat equation (J. Appl. Physiol. vol. 1, (1948), 93-122)

$$\frac{\delta T}{\delta t} r_t C_t + w_b c_b (T - T_a) = k \triangledown^2 T + (Q_p + Q_m)$$

where $r_t$ (kg/m$^3$) is the density of tissue, $C_t$ (J/kg°C) is the specific heat of tissue, t (s) is the time, T (°C) is the temperature, $w_b$ (kg/m$^3$s) is the blood perfusion, $c_b$ (J/kg °C) is the specific heat of blood, $T_a$ (°C) is the arterial temperature, k (W/m °C) is the thermal conductivity of tissue, $Q_p$ (W/m$^3$) is the power deposition and $Q_m$ (W/m$^3$) is the local metabolic rate. Hence, the thermosensitive particulate compositions may, after a controlled, localized external heating, give a measure of blood perfusion in an organ.

The temperature response of thermosensitive MR-liposomes can in general be divided into three distinct regions:

a) 'low relaxivity' region; $r_1 = r_1^{low}$; $T < T_a$; where $r_1^{low}$ is a constant with temperature (T);
b) 'temperature active' region' $r_1(T) = f(T)$, $T_a < T < T_b$, and
c) 'high relaxivity' region; $r_1 = r_1^{high}$; $T > T_b$; where $r_1^{high}$ is a constant with T (ideally, $r_1^{high} >> r_1^{low}$).

It is possible to quantify the local temperature in the temperature active region of the liposomes, provided three criteria are met:

1. A well defined relationship exists around T between liposomal relaxivity and temperature; i.e. $r_1(T) = f(T)$; $T_a < T < T_b$ ; where $T_a$;$T_b$ is a clinically relevant temperature range. Ideally $r_1$ should be a linear function of T over the range $T_a$;$T_b$.
2. The temperature active region covers a large enough temperature range.
3. The Gd concentration in tissue [Gd] is known.

If [Gd] is not known, even a qualitative assessment of temperature changes may prove difficult, since regions with different [Gd] would have a different degree of enhancement, even if the temperature were the same. Furthermore, it would be impossible to say whether lack of enhancement after heating was due to a low local temperature or the absence of the liposomes in that region.

However the local [Gd] *in vivo* can be estimated, based on the relaxation effects of the liposomes in the 'low relaxivity' state, by the following method:

1. Acquire quantitative $R_1$ and/or $R_2/R_2^*$ images ($R_{1,2}=1/T_{1,2}$) before contrast administration and after contrast administration but before hyperthermia is initiated. $R_1$ and $R_2/R_2^*$ images can be routinely acquired on most state-of-the-art clinical MR systems.
2. Measure the fractional change in $R_1$ and/or $R_2/R_2^*$, $\Delta R_1=R_1^{post}-R_1^{pre}$ in the region of interest.
3. The local Gd concentration is then given by: $[Gd] = \Delta R_1/r_1$.
4. If r, is not known, the ratio of the Gd concentration between two regions is given by: $[Gd.] / [Gd_2] =) R_1 /\Delta R1$. Alternatively, $R_2$ or $R_2^*$ images can be used to obtain the same Gd ratio.

In general therefore, the absolute [Gd] can not be determined, unless the liposomal relaxivity in the tissue is known. However this may be fairly well approximated for the r relaxivity, but not the r * relaxivity, since this depends on tissue geometry. Nonetheless, the [Gd] in one region relative to another can be estimated as described above. The relative [Gd] is valuable information that can be used to adjust the signal enhancement in the image so that it reflects actual temperature changes. In order for this to be possible, one need to assume that a 'core region' exists where the temperature is above T. It is likely in a clinical situation that such a core region exists where the heating is most efficient surrounded by a 'penumbra' where heating is less efficient and the temperature distribution is less well defined. Now, by knowing the relative [Gd] in the core versus the penumbra, the image intensity can be adjusted to compensate for any difference in [Gd] in the two regions.

It is possible to estimate $[Gd_1]/[Gd_2]$ using a strongly $T_1$-weighted sequence in which case change in signal intensity is almost linearly related to change in $R_1$ and hence [Gd]. This requires $TR \ll T_1$ of the target tissue. Similarly, strongly $T_2$ or $T_2^*$-weighted sequences can be used to estimate $[Gd_1] / [Gd_2]$ .

Thus, when a Gd compound is encapsulated in liposomes, the resulting relaxivity $(r_1,r_2)$ is small due to restricted water access to the paramagnetic centre. However, given that a very $T_1$-sensitive sequence is used, it is still possible to detect a change in $T_1$ due to the presence of the liposomes prior to heating (i.e. at temperatures well below $T_0$). Consequently, by acquiring quantitative $R_1$- or $T_1$-maps of the area of interest before and after contrast injection (prior to hyperthermia treatment), the change in $R_1$ or $T_1$ enables the local Gd concentration [Gd] to be determined. After heating, regional variations in [Gd] can thus be accounted for; variations in contrast enhancement due to temperature differences can therefore be distinguished from variations in contrast enhancement due to concentration variations.

The longitudinal relaxation rate $R_1$ after contrast administration is given by:

$$R_1 = R_1^0 + [Gd]^*r_1;$$

Where $R_1^0$ is the relaxation rate prior to contrast administration. The change in $R_1$ due to the contrast agent is therefore:

$$\Delta R_1 = R_1 - R_1^0 = [Gd]^*r_1;$$

After hyperthermia, a new $R_1$- or $T_1$-map is generated.

In conclusion, therefore, given that the $T_1$ effect of the liposomes is detectable below $T_0$, it is possible to map the local Gd concentration and consequently compensate for differences in contrast enhancement after heating due to local variations in Gd concentration. The $R_2$ or $R_2'$ effect of the liposomes can also be used for this purpose.

In vivo pH measurements have been of great interest because pH is an important physiological parameter associated to several severe diseases. The pH value is usually reduced during cancer diseases, cardiovascular diseases like for example stroke, osteoporosis, inflammations and autoimmune diseases.

One type of pH sensitive encapsulation for diagnostic agents involves the use of pH sensitive liposomes. The general strategy is to employ pH-sensitive groups in the liposomal membrane. Such typical groups have pKa values between 4 and 5.5. Phospholipids useful for preparation of pH-sensitive diagnostic agents include diheptadecanoyl phosphatidylcholine (DHPC) in admixture with DPPC and N-palmitoyl homocystein (PHC) in different ratios (see Eur. J. Pharm. Biopharm. 1993, 39, 97-101 for a general review on temperature and pH-sensitive liposomes).

Another type of pH-sensitive encapsulation of contrast generating species involves the use of pH-sensitive surfactants like for example N-dodecyl-2-imidazole propionate (DIP) which has pKa of 6.8 (see for example Pharm. Res. 1993, 13, 404). This means that DIP at pH 7.3-7.4 (physiological) is in the non-ionized (non/low surfactant activity) form (80%) while at for example lysosomal pH (5.2) over 97% will be in the charged form.

Another means of pH-sensitive encapsulation of magnetic resonance contrast generating species involves the use of matrix materials and/or coating materials with pKa values in the range of 4.5-7.0 so that the material is soluble or partly soluble in the charged form and insoluble or partly insoluble in the non-charged form. Such compounds can be physiologically acceptable low molecular weight compounds or physiologically acceptable polymers.

Still another means of pH-sensitive encapsulation involves the use of compounds that are chemically cleaved as a result of pH, for example polyorthoesters or polyacetals/ketals which are cleaved under acidic conditions.

Liposomes comprising phosphatidyl ethanolamines (PE) as the central component are another example of liposomes which can undergo a phase transition and become leaky when pH is reduced. pH sensitive liposomes can also be achieved by incorporation of fatty acids into phospholipid membranes.

In principle any charged particulate system where the charge is pH dependent and influences the packing of the membrane material can be used.

Access to oxygen is critical for all types of cells, and diagnostic agents for determination of oxygen concentration/tension in tissue will be of great importance in diagnosis of diseases like cancer, cardiovascular diseases, autoimmune diseases and several diseases in the central nervous system.

One type of oxygen or redox sensitive encapsulation/coating material is a material that has different solubility/diffusion properties dependent on the oxygen level or the redox status; for example compounds containing a nitrogroup that is reduced in vivo to an amino-group which improves solubilization of the material in reductive/low oxygen surroundings.

Determination of concentration of physiologically important ions in tissue is important for several diseases.

Types of ion concentration sensitive encapsulation materials that may be used in this regard include phospholipids, surfactants and other ion chelating materials. Negatively charged liposomes will for example bind Ca(2+) and the membrane will change its diffusion properties and become more stiff.

An example of $Ca^{2+}/Mg^{2+}$ sensitive particulate compositions are liposomes enriched with the dimeric phospholipid cardiolipin. A cardiolipin containing membrane may undergo a lamellar to reversed hexagonal phase transition upon addition of the divalent cations since these ions bind to the cardiolipin di-phosphatidyl group.

$Ca^{2+}$ or $Mg^{2+}$ sensitivity may be obtained by using charge stabilised particles, e.g. solid particles, liquid particles e.g. emulsion droplets, gas particles e.g. microbubble dispersions or liposomes. $Ca^{2+}$ or $Mg^{2+}$ may thus induce aggregation or flocculation among the particles and by this means alter contrast effect. $Ca^{2+}$ or $Mg^{2+}$ sensitivity may also be obtained by using stabilising moieties for the particles which are chemically or physically influenced by $Ca^{2+}$ or $Mg^{2+}$, for instance using surfactants which form water insoluble species when exposed to $Ca^{2+}$ or $Mg^{2+}$ and thus precipitate.

Some particles or stabilising membranes surrounding particles may also respond with a phase transition when exposed to $Ca^{2+}$ or $Mg^{2+}$. An example are liquid crystalline based particles e.g. liposomes, which may respond by a lamellar to reversed hexagonal phase transition upon addition of $Ca^{2+}$ or $Mg^{2+}$. Also gel particles may respond easily to $Ca^{2+}$ or $Mg^{2+}$ by a significant lowering of viscosity or even phase separation of the polymer which forms basis for the gel on exposure to $Ca^{2+}$ or $Mg^{2+}$. This viscosity reduction or phase separation may induce a change in contrast effect.

Types of enzyme sensitive encapsulation material include matrices or coatings that are degraded by enzymes, for example simple esters of low molecular weight compounds or polyesters like polyacetic acid and others.

Various metabolites may also change the properties of coating materials.

Particulates can be made sensitive to for example antibodies based on enhanced leakage due to a phase transition induced by the chemical binding between membrane molecules and the antibody. As an example, liposomes comprising N-(dinitrophenylamino-ε-caproyl)-phosphatidyl ethanolamine (DNP-cap-PE) become leaky due to a lamellar to reversed hexagonal phase transition when binding to anti-DNP. Another example includes liposomes comprising human glycophorin A in dioleoyl phosphatidyl ethanolamine membranes. These liposomes become leaky when immobilized antibodies are added.

A further aspect of the present invention is to use one of the above described particulate diagnostic agents together with another compound that has the potential to change the physiological parameter of interest or together with use of an external energy source to change the parameter of interest.

Thus one example is to administer thermosensitive MR diagnostic agents according to the invention in connection with an external heating and to follow the heating effect in parts of the body.

Another example is to administer compounds that change pH in connection with a pH-sensitive particulate MR diagnostic agent according to the invention to follow the pH-profile in the area of interest.

Still another example is to cause the subject under study to inhale oxygen, after administration of an oxygen sensitive MR diagnostic agent according to the invention, to follow oxygen uptake in tissue.

Early diagnosis is very important to obtain good therapeutic results. In most disease processes changes in physiological parameters take place before changes in morphology. All existing contrast agents diagnose morphology. The new types of contrast agent according to the invention are able to detect diseases at a very early stage in the disease process and thereby improve the therapeutic outcome for the patient.

Where the particulate diagnostic agent or a component thereof carries an overall charge, it will conveniently be used in the form of a salt with a physiologically acceptable counterion, for example an ammonium, substituted ammonium, alkali metal or alkaline earth metal cation or an anion deriving from an inorganic or organic acid. In this regard, meglumine salts are particularly preferred.

The diagnostic agents of the present invention may be formulated in conventional pharmaceutical or veterinary parenteral administration forms, e.g. suspensions, dispersions, etc., for example in an aqueous vehicle such as water for injections.

Such compositions may further contain pharmaceutically acceptable diluents and excipients and formulation aids, for example stabilizers, antioxidants, osmolality adjusting agents, buffers, pH adjusting agents, etc.

Where the agent is formulated in a ready-to-use form for parenteral administration, the carrier medium is preferably isotonic or somewhat hypertonic.

Where the particulate agent comprises a chelate or salt of an otherwise toxic metal species, e.g. a heavy metal ion, it may be desirable to include within the formulation a slight excess of a chelating agent, e.g. as discussed by Schering in DE-A-3640708, or more preferably a slight excess of the calcium salt of such a chelating agent.

The dosage of the diagnostic agents of the invention will depend upon the imaging modality, the contrast generating species and the means by which contrast enhancement occurs (e.g. with switching on or off of contrast, with dispersion of contrast out of the vascular space, etc).

In general however dosages will be between 1/10 and 10 times the dosage conventionally used for the selected contrast generating species or analogous species in the same imaging modality. Even lower doses may also be used.

While the present invention is particularly suitable for methods involving parenteral administration of the particulate material, e.g. into the vasculature or directly into an organ or muscle tissue, intravenous administration being especially preferred, it is also applicable where administration is not via a parenteral route, e.g. where administration is transdermal, nasal, sub-lingual or is into an externally voiding body cavity, e.g. the gi tract, the bladder, the uterus or the vagina. The present invention is deemed to extend to cover such administration.

The disclosures of all the documents mentioned herein are incorporated by reference.

The present invention will now be illustrated further by reference to the following non-limiting

Examples.

### Example 1

Preparation of Temperature Sensitive Paramagnetic Liposomes

Liposomes containing GdHPDO3A (ProHance, Bracco Spa, Milan, Italy) and GdDTPA-BMA (Omniscan, Nycomed Amersham Imaging AS, Oslo, Norway) were prepared by the thin film hydration method. Two different saturated phospholipid blends were used; one consisting of hydrogenated phosphatidyl choline (HPC) (Lipoid GmbH, Ludwigshafen, Germany) and hydrogenated phosphatidylserine-sodium (HPS) (NOF Corporation, Amagasaki, Japan); the other composed of DPPC and DPPG-sodium (Sygena Ltd, Liestal, Switzerland). The phospholipid mixtures contained 5% or 10% (w/w) of the negatively charged HPS and DPPG components. The phospholipid mixtures were dissolved in a chloroform/ methanol mixture and the organic solution was evaporated to dryness under reduced pressure. DPPC/ DPPG liposomes were formed by hydrating the lipid film with a pre-heated (55°C) aqueous solution (pH 7.4) of 250 mM GdDTPA-BMA or 250 mM Gd HPD03A. The HPC/HPS liposomes were prepared analogously but with a lipid hydration temperature of 70°C. The DPPC/DPPG and HPC/HPS liposomes where allowed to swell for 2 hours at 55 and 70°C respectively. The total lipid concentration was 50 mg/ml. The liposomes were subjected to 3 freeze-thaw cycles in liquid nitrogen. Differently sized liposomes were produced by sequential extrusion (Lipex Extruder™, Lipex Biomembranes Inc., Vancouver, Canada) through polycarbonate filters of various pore diameters. The extrusion temperature was 55 and 70°C for the DPPC/DPPG and HPC/HPS liposomes respectively. Untrapped metal chelate was removed by gel filtration or dialysis against isoosmotic and isoprotic glucose solution.

Physiochemical Properties

The mean hydrodynamic diameter of the liposomes varied from 103 nm to 276 nm, as measured by photon correlation spectroscopy (ZetaSizer IV, Malvern Instruments Ltd., Malvern, England); the zeta potential was negative in the order of -25 mV, as determined by laser Doppler velocimetry at 25°C (ZetaSizer IV, Malvern Instruments Ltd., Malvern, England). The mean gel-to-liquid crystalline phase transition temperature (Tc) of the HPC/HPS and DPPC/ DPPG preparations was 50 and 42°C, respectively, as determined by differential scanning calorimetry (DSC4, Perkin Elmer Inc., Norwalk, CT).

Temperature Response of *In Vitro* MR Contrast Efficacy

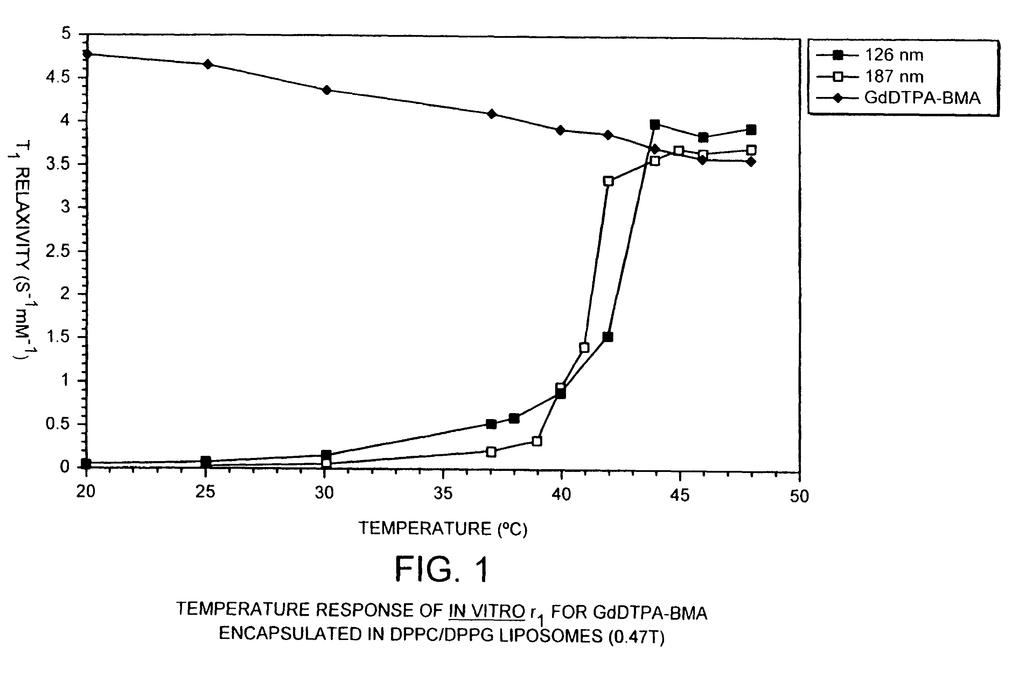

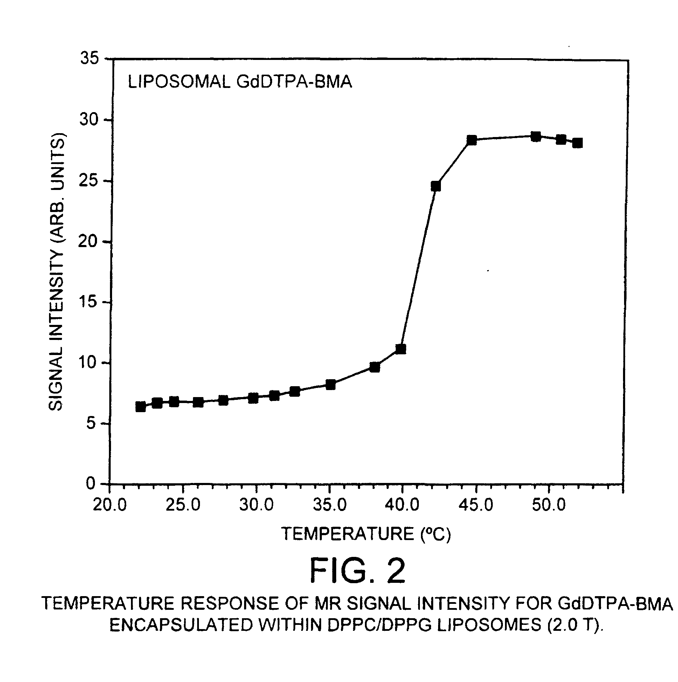

Figure 1 of the accompanying drawings and Table 5 below show the temperature sensitivity of in vitro $T_1$ relaxivity ($r_1$) for liposome encapsulated GdDTPA-BMA and GdHPDO3A, respectively (0.47T). Figure 2 of the accompanying drawings shows the temperature response of the in vitro MR signal intensity for liposome encapsulated Gd-DTPA-BMA.

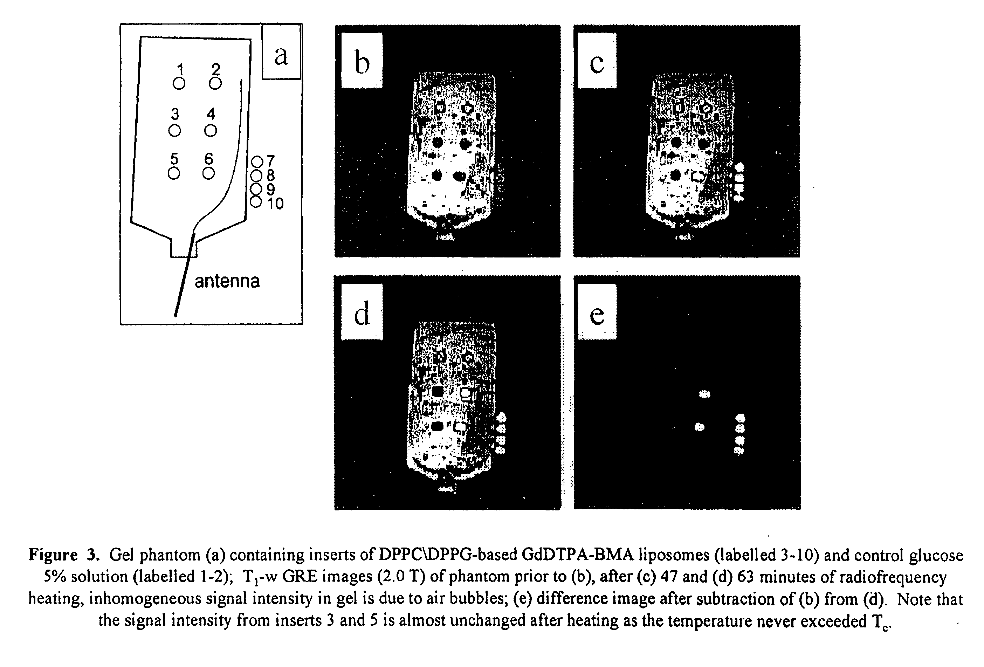

Figure 3 of the accompanying drawings shows a series of $T_1$-w GRE images prior to and after heating of a gel phantom containing inserts of liposome encapsulated GdDTPA-BMA.

Table 5

| Temperature (°C) | $r_1$ ($s^{-1}$ $mM^{-1}$) | | |
|---|---|---|---|
| | DPPC/DPPG 103 nm | HPC/HPS 130 nm | Control* |
| 20 | 0.16 | 0.06 | 4.53 |
| 25 | 0.23 | 0.08 | 4.27 |
| 30 | 0.31 | 0.12 | 3.94 |
| 37 | 0.69 | 0.21 | 3.75 |
| 45 | 3.30 | 0.53 | 3.07 |
| 55 | 3.10 | 3.00 | 2.82 |
| 60 | - | 2.96 | 2.54 |

\* non-liposomal GdHPDO3A

**Example 2**

GdDTPA-BMA encapsulated within DSPC/DPPC/DPPG liposomes

DSPC/DPPC/DPPG (weight ratio; 28.5/66.5/5) liposomes were prepared by the thin film hydration method. The phospholipids (500 mg) were dissolved in a chloroform/methanol mixture and the organic solution was evaporated to dryness under reduced pressure. Liposomes were formed by hydrating the lipid film with a pre-heated (57"C) aqueous solution (pH ≃ 7) of 250 mM GdDTPA-BMA (10 ml). The liposomes were subjected to 3 freeze-thaw cycles and allowed to swell for one and a half hours at 65"C. The liposome dispersion was extruded at 65°C through polycarbonate filters of various pore diameters. The liposome size (z-average) after extrusion was 167 nm. Untrapped GdDTPA-BMA was removed by dialysis against isoosmotic and isoprotic glucose solution.
*Table 6* shows the temperature sensitivity of the *in vitro* $r_1$ (0.235T) in glucose 5% solution for liposome encapsulated GdDTPA-BMA.

Table 6

| Temperature ( °C) | $r_1$ in glucose 5% ($s^{-1}mM^{-1}$) |
|---|---|
| 30 | 0.098 |
| 35 | 0.13 |
| 38 | 0.22 |
| 40 | 0.27 |
| 41 | 0.31 |
| 43 | 1.10 |
| 45 | 2.92 |

**Example 3**

GdDTPA-BMA encapsulated within DPPC/DPPG/DPPE-PEG-2000 liposomes

DPPC/DPPG/DPPE-PEG-2000 (weight ratio; 90/5/5) liposomes were prepared by the thin film hydration method. The phospholipids (500 mg) were dissolved in a chloroform/methanol mixture and the organic solution was evaporated to dryness under reduced pressure. Liposomes were formed by hydrating the lipid film with a pre-heated (57 C) aqueous solution (pH ≃ 7) of 250 mM GdDTPA-BMA (10 ml). The liposomes were subjected to 3 freeze-thaw cycles and allowed to swell for one and a half hours at 65°C. The liposome dispersion was extruded at 65°C through polycarbonate filters of various pore diameters. The liposome size (z-average) after extrusion was 132 nm. Untrapped GdDTPA-BMA was removed by dialysis against isoosmotic and isoprotic glucose solution.

*Table* 7 shows the temperature sensitivity of the in *vitro* r (0.235T) in glucose 5% solution for liposome encapsulated GdDTPA-BMA.

Table 7

| Temperature (°C) | $r_1$ in glucose 5% ($s^{-1}mM^{-1}$) |
|---|---|
| 35 | 0.32 |
| 37 | 0.46 |
| 38 | 0.56 |
| 39.2 | 2.53 |
| 40 | 4.16 |
| 42 | 5.65 |

## Example 4

GdDTPA-BMA encapsulated within DSPC/DPPC/DPPG liposomes

DSPC/DPPC/DPPG (weight ratio; 43/52/5) liposomes were prepared by the thin film hydration method. The phospholipids (500 mg) were dissolved in a chloroform/methanol mixture and the organic solution was evaporated to dryness under reduced pressure. Liposomes were formed by hydrating the lipid film with a pre-heated (63"C) aqueous solution (pH ≃ 7) of 250 mM GdDTPA-BMA (10 ml). The liposomes were subjected to 3 freeze-thaw cycles and allowed to swell for one and a half hours at 64 C. The liposome dispersion was extruded at 65 C through polycarbonate filters of various pore diameters. The liposome size (z-average) was 145 nm. Untrapped metal chelate was removed by dialysis against isoosmotic and isoprotic glucose solution.

*Table 8* shows the temperature sensitivity of the in *vitro* r (0.235T) in both glucose 5% solution and human serum for liposome encapsulated GdDTPA-BMA.

Table 8

| Temperature (°C) | $r_1$ in glucose 5% ($s^{-1}mM^{-1}$) | $r_1$ in serum ($s^{-1}mM^{-1}$) |
|---|---|---|
| 35 | 0.12 | 0.14 |
| 40 | 0.22 | 0.25 |
| 42 | 0.29 | 0.44 |
| 44 | 0.88 | 1.91 |
| 46 | 4.47 | 4.51 |
| 48 | 4.40 | 4.51 |
| 50 | 4.40 | 4.35 |

## Example 5

GdDTPA-BMA encapsulated within DPPC/DPPG liposomes

DPPC/DPPG (weight ratio; 95/5) liposomes were prepared by the thin film hydration method. The phospholipids (500 mg) were dissolved in a chloroform/methanol mixture and the organic solution was evaporated to dryness under reduced pressure. Liposomes were formed by hydrating the lipid film with a pre-heated (52°C) aqueous solution (pH ≃ 7) of 250 mM GdDTPA-BMA (10 ml). The liposomes were subjected to 3 freeze-thaw cycles and allowed to swell for one and a half hours at 55°C. The liposome dispersion was extruded at 62"C through polycarbonate filters of various pore diameters. The liposome size (z-average) after extrusion was 148 nm. Untrapped metal chelate was removed by dialysis against isoosmotic and isoprotic glucose solution.

*Table 9* shows the temperature sensitivity of the *in vitro* $r_1$ (0.235 T) in both glucose 5% solution and human serum for liposome encapsulated GdDTPA-BMA.

Table 9

| Temperature (°C) | $r_1$ in glucose 5% ($s^{-1}mM^{-1}$) | $r_1$ in serum ($s^{-1}mM^{-1}$) |
|---|---|---|
| 35 | 0.331 | 0.389 |
| 38 | 0.753 | 0.810 |
| 39 | 1.47 | 1.20 |
| 40 | 3.75 | 3.31 |
| 41 | 4.88 | 5.05 |
| 42 | 4.80 | 4.99 |
| 44 | 4.80 | 4.78 |
| 48 | 4.77 | 4.88 |

**Example 6**

"Double transition" with a mixture of DSPC/DPPC/DPPG and DPPC/DPPG liposomes, containing both GdDTPA-BMA

1.5 ml liposomes from Example 4 were mixed with 1.5 ml DPPC/DPPG liposomes prepared as Example 5. The mixture was diluted to 40 ml with glucose 5% solution.

*Table 10* shows the temperature sensitivity of the *in vitro* $R_1$ (0.235 T) in glucose 5% solution for the liposome mixture.

Table 10

| Temperature ( C) | R in glucose 5% ($s^-$ ) |
|---|---|
| 35 | 2.46 |
| 38 | 2.61 |
| 39 | 2.83 |
| 40 | 3.87 |
| 41 | 7.11 |
| 42 | 7.17 |
| 44 | 10.9 |
| 46 | 14.0 |
| 48 | 14.0 |

**Example 8**

Imaging studies in rats with GdDTPA-BMA encapsulated within DPPC/DPPG liposomes

a) Intramuscular injection in the left thigh

Liposomes were injected intramuscularly at a dosage of 0.02 mmol/kg. The left thigh muscle was heated with focused ultrasound whereas the right thigh muscle served as a control.

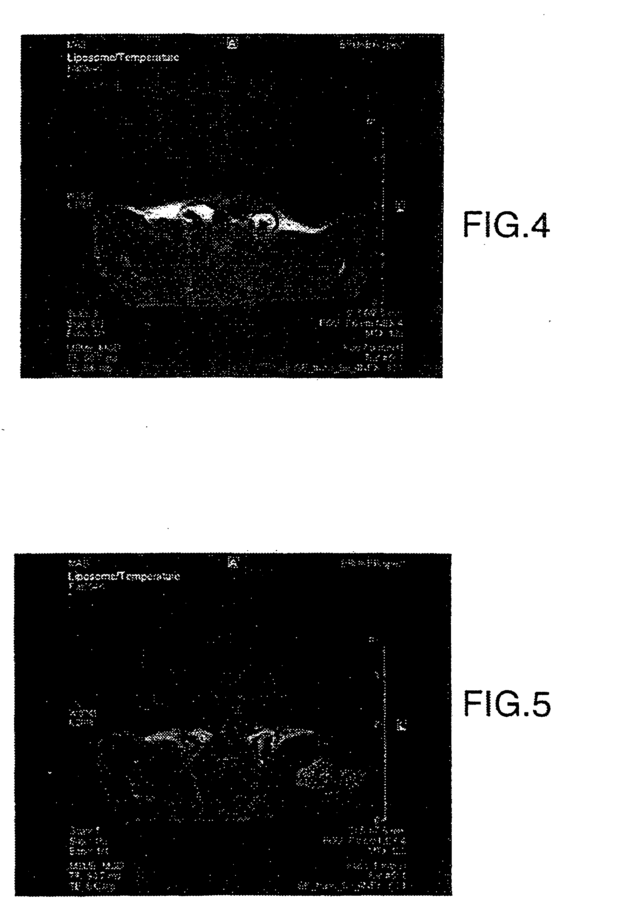

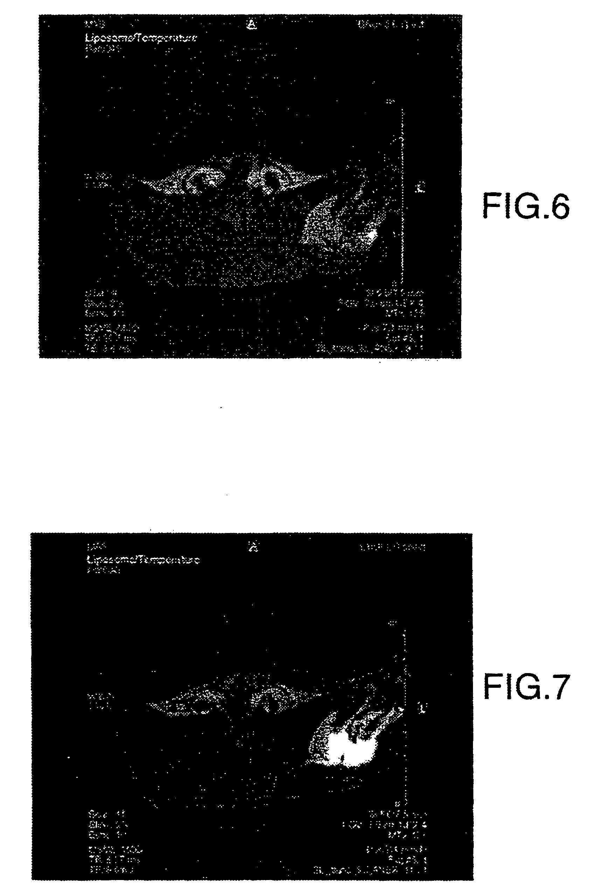

Figures 4-5 show axial $T_1$-w SE images of the thigh before and after liposome injection, respectively. Figures 6-8 are $T_1$-w SE images after 2, 5, and 9 minutes of heating, respectively.

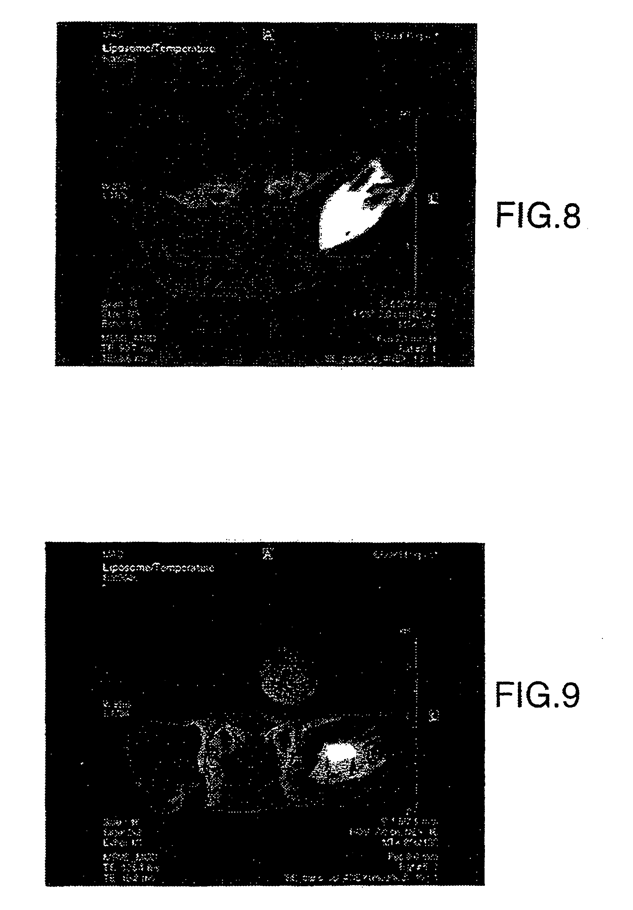

At that timepoint, heating was terminated, the rat was removed from the MRI scanner and the temperature of the muscle was measured to be 47°C. Figure 9 represents the final image 15 minutes after termination of heating. For comparative purposes, the syringe containing the liposomal dispersion (identical to that injected) was included.

The results indicate that the signal intensity of the left thigh muscle increases substantially after heating, as compared to the right thigh muscle and syringe.

b) Intravenous injection

Liposomes were injected intravenously into a rat (upper position) at a dosage of 0.10 mmol/kg. The rat in the lower position served as control (e.g. no injection nor heating).

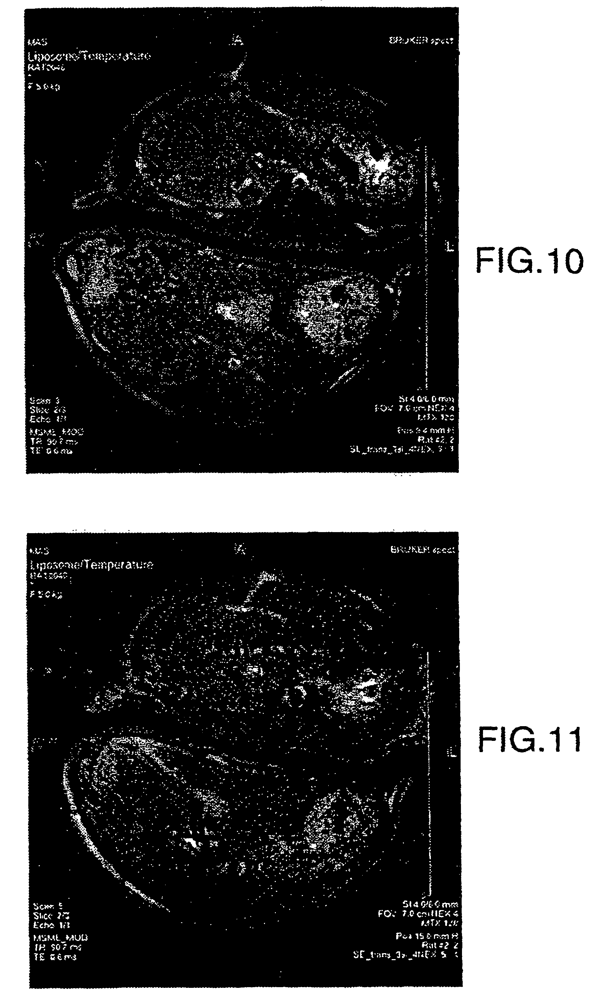

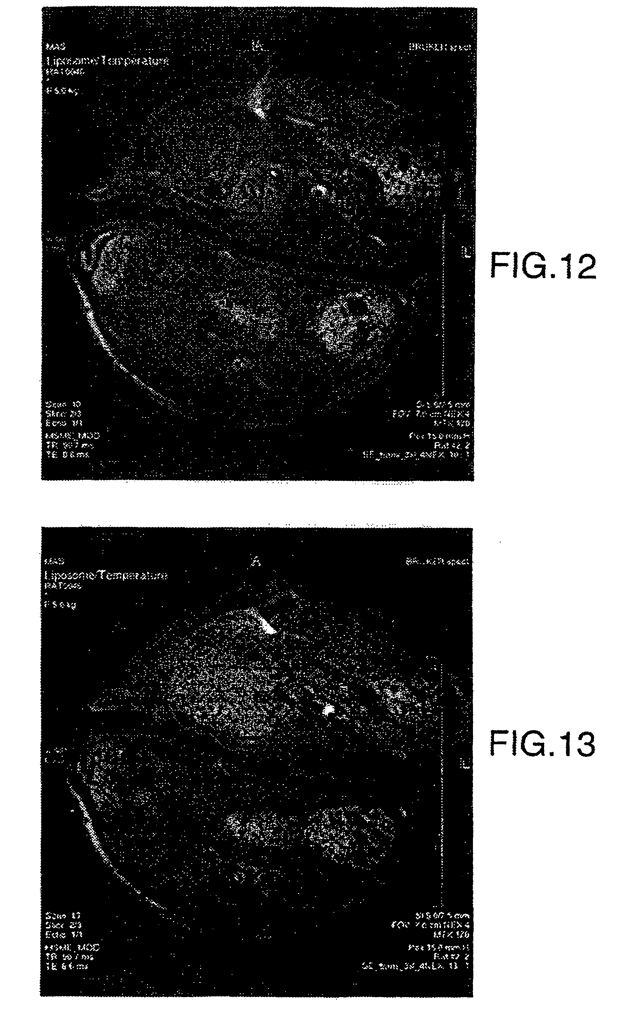

Figure 10 is the axial $T_1$-w SE image of the liver 7 minutes after liposome injection. At 15 minutes post injection, the liver was heated by focused ultrasound (Figure 11). Figures 12-13 are $T_1$-w SE images 16 and 21 minutes after initiation of heating, respectively. After termination of heating, the measured temperature in the liver was 51°C.

The results indicate that the liver signal intensity increases substantially after heating as compared to the control liver.

**Example 9**

Preparation of pH-sensitive paramagnetic liposomes

Liposomes composed of DPPE/PA (4:1 mol/mol) containing GdDTPA-BMA were prepared by the thin film hydration method. The total lipid concentration was 25 mg/ml. Briefly, a chloroform/methanol (10:1) solution of the lipids was rotary evaporated to dryness and the resulting film was further dried under vacuum over night. The lipids were hydrated with 250 mM GdDTPA-BMA in 0.05 M Tris-HCl buffer (pH = 8.4) at 75 °C. The liposomes were subjected to 3 freeze-thaw cycles in MeOH/$CO_2$(s). The liposomes were sized down by sequential extrusion (Lipex Extruder™, Lipex Biomembranes Inc., Vancouver, Canada) through polycarbonate filters with various pore diameters. Untrapped metal chelate was removed by dialysis against isoosmotic glucose solution (pH=8.4).

Physicochemical properties

The mean hydrodynamic diameter of the liposomes was measured to 165 nm by photon correlation spectroscopy (ZetaSizer IV, Malvern Instruments Ltd., Malvern, England). The *in vitro* $T_1$-relaxation times of the paramagnetic liposomes were measured (0.235 T, Minispec PC-110b, Bruker GmbH, Rheinstetten, Germany) in different isoosmotic buffer solutions (0.05 M citrate-phosphate buffer and 0.05 M Tris-HCl buffer). The investigated pH range was 4-8.5. The buffered liposome dispersions were incubated at 37°C for 15 minutes.

Table 11 shows the pH sensitivity of *in vitro* $r_1$-relaxivity for liposome encapsulated GdDTPA-BMA.

Table 11

| pH dependency of the $r_1$ (37 °C, 0.235 T) for liposomal GdDTPA-BMA | |
|---|---|
| pH | $r_1$ ($s^{-1}$ $mM^{-1}$) |
| 3.91 | 1.32 |
| 4.30 | 1.26 |
| 4.70 | 1.31 |
| 5.15 | 1.17 |
| 5.59 | 1.10 |
| 5.95 | 1.03 |
| 6.40 | 1.00 |
| 6.71 | 0.50 |
| 7.33 | 0.32 |
| 7.69 | 0.29 |
| 8.02 | 0.28 |
| 8.34 | 0.29 |
| 8.54 | 0.31 |

**Example 10**

DyDTPA-BMA encapsulated within DPPC/DPPG liposomes

DPPC/DPPG (weight ratio; 95/5) liposomes were prepared by the thin film hydration method. The phospholipids (500mg) were dissolved in a chloroform/methanol mixture and the organic solution was evaporated to dryness under reduced pressure. Liposomes were formed by hydrating the lipid film at 50°C with an aqueous solution (pH ≃ 7) of 250 mM DyDTPA-BMA (sprodiamide, Nycomed Imaging AS, Oslo, Norway) (10ml). The liposomes were subjected to 3 freeze-thaw cycles and allowed to swell for one hour at 59°C. The liposome dispersion was extruded at 65°C through polycarbonate filters of various pore diameters. The liposome size (z-average) after extrusion was 153 nm. Untrapped DyDTPA-BMA was removed by dialysis against isoosmotic and isoprotic glucose solution. The temperature sensitivity of the MR contrast effect may be investigated.

**Example 11**

GdDTPA-dextran encapsulated within DPPC/DPPG liposomes

DPPC/DPPG (weight ratio; 95/5) liposomes were prepared by the thin film hydration method. The phospholipids (500 mg) were dissolved in a chloroform/methanol mixture and the organic solution was evaporated to dryness under reduced pressure. The liposomes were formed by hydrating the lipid film at 48°C with an aqueous solution of 50 mM GdDTPA-dextran (MW 156 kD), whose synthesis is described in: *P Rongved et al., Carbohydr. Res*., *287 (1996)* 77-89. The liposome dispersion was sonicated at 46°C using a sonicator tip. The liposome size (z-average) after sonication was 70 nm. Untrapped GdDTPA-dextran is removed by gel filtration or dialysis against isoosmotic and isoprotic glucose solution. The temperature sensitivity of the MR contrast effect may be investigated.

**Example 12**

GdDTPA-BMA encapsulated within dibehenoyl-PC liposomes

Dibehenoyl-PC (22:0) (Table 1) liposomes may be prepared by the thin film hydration method. The phospholipids (500 mg) are dissolved in a chloroform/methanol mixture and the organic solution is evaporated to dryness under reduced pressure. Liposomes are formed by hydrating the lipid film at 80°C with an aqueous solution (pH ≃ 7) of 250 mM GdDTPA-BMA (10 ml). The liposomes are subjected to 3 freeze-thaw cycles and allowed to swell for one and half-hours at 80°C. The liposome dispersion is extruded at 80°C through polycarbonate filters of various pore diameters. Untrapped GdDTPA-BMA is removed by gel filtration or dialysis against isoosmotic and isoprotic glucose solution. The temperature sensitivity of the MR contrast effect may be investigated.

**Example 13**

Superparamagnetic iron oxides (SPIOs) encapsulated within HPC/HPS liposomes

HPC/HPS (weight ratio; 90/10) liposomes were prepared by a modified thin film hydration method. Liposomes were formed by adding a homogeneous mixture of phospholipids (700 mg) to 10 ml of a pre-heated (55°C) aqueous dispersion of PEGylated SPIOs (6.10 mg iron/ml). The liposomes were allowed to swell for 30 minutes at 65°C. The liposome dispersion was extruded at 66°C through polycarbonate filters of various pore diameters. Untrapped SPIOs are removed by gel filtration or dialysis. The temperature sensitivity of the MR contrast effect may be investigated.

**Example 14**

Ultrasmall superparamagnetic iron oxides (USPIOs) encapsulated within HPC/HPS liposomes

HPC/HPS (weight ratio; 90/10) liposomes were prepared by a modified thin film hydration method. Liposomes were formed by adding a homogeneous mixture of phospholipids (700 mg) to 10 ml of a pre-heated (70°C) aqueous dispersion of USPIOs (3.63 mg iron/ml). The liposomes were allowed to swell for 90 minutes at 70°C. The liposome dispersion was extruded at 70°C through polycarbonate filters of various pore diameters. Untrapped USPIOs are removed by gel filtration or dialysis. The temperature sensitivity of the MR contrast effect may be investigated.

**Example 15**

Superparamagnetic iron oxides (SPIOs) or ultrasmall-SPIOs encapsulated within pH-sensitive liposomes

SPIOs or USPIOs encapsulated within pH-sensitive liposomes may be prepared in a manner analogous to that used in Example 9. Untrapped superparamagnetic material is removed by gel filtration or dialysis. The pH-sensitivity of the MR contrast effect may be investigated.

**Example 16**

GdDTPA-BMA encapsulated within DSPC/DMPG/cholesterol liposomes

DSPC/DMPG/cholesterol liposomes (molar ratio; 49:5:20) were prepared by a modified thin film hydration method. Liposomes were formed by adding a freeze-dried mixture of phospholipids (60 g) to a pre-heated (59°C) aqueous solution (pH $\cong$ 6.3) of 250 mM GdDTPA-BMA/300 mM sucrose/10mM phosphate (300 ml). The liposomes were allowed to swell for 30 minutes at 59°C. The liposome dispersion was homogenized and extruded at high pressure through polycarbonate filters with a pore size of 400 nm. Untrapped GdDTPA-BMA was removed by ultrafiltration with a 300 mM sucrose/10 mM phosphate solution. The liposome size (z-average) after ultrafiltration was 110 nm. Liposomes were also lyophilized (2ml per vial) and reconstituted by addition of 2 ml of deionized water. The liposome size (z-average) after reconstitution was 119 nm.

Table 12 summarizes the temperature sensitivity of the *in vitro* $R_1$ (0.235 T) for liposome encapsulated GdDTPA-BMA in a 300 mM sucrose/10 mM phosphate solution. The influence of lyophilization on the $R_1$-temperature sensitivity of the reconstituted liposomes is also shown in *Table 12*.

Table 12

|  | $T_1$ Relaxation Time (ms) | | |
|---|---|---|---|
|  | 35°C | 40°C | 55°C |
| Before lyophilisation | 930 | 750 | 350 |
| After lyophilisation | 670 | 590 | 340 |

**Example 17**

Commercially available Gd compounds/Gd compounds in development phase encapsulated within temperature- or pH-sensitive liposomes

Liposomes containing the following contrast agents: GdBOPTA (Bracco spa, Italy), GdDTPA (Shering AG, Berlin), GdDOTA (Guerbet SA, Aulnay-sous-Bois), Gadomer (Shering AG, Berlin) MS-325 and protein bound MS-325 (Epix Medical Inc, USA) may be prepared in a manner analogous to that used in Examples 1-5, 9 and 12. Untrapped Gd compound is removed by gel filtration or dialysis. The temperature sensitivity of the MR contrast effect may be investigated.

**Example 18**

Temperature sensitivity of *in vitro* $r_1$ in blood for liposome encapsulated GdDTPA-BMA

DPPC/DPPG/DPPE-PEG-2000 and DPPC/DPPG liposomes containing GdDTPA-BMA were prepared in a manner analogous to that used in Examples 3 and 5, respectively. Table 13 summarizes the temperature evolution of the *in vitro* $r_1$ (0.235 T) in rat blood for both liposome formulations.

Table 13

| Temperature (°C) | $r_1$ ($s^{-1}mM^{-1}$) DPPC/DPPG 120 nm | $r_1$ ($s^{-1}mM^{-1}$) DPPC/DPPG/DPPE-PEG 121 nm |
|---|---|---|
| 35 | 0.260 | 0.244 |
| 37 | 0.479 | 0.391 |
| 39 | 0.659 | 0.588 |

Table 13 (continued)

| Temperature (°C) | $r_1$ (s⁻¹mM⁻¹) DPPC/DPPG 120 nm | $r_1$ (s⁻¹mM⁻¹) DPPC/DPPG/DPPE-PEG 121 nm |
|---|---|---|
| 40 | 1.18 | 0.823 |
| 41 | 2.45 | 1.26 |
| 42 | 4.06 | 2.87 |
| 43 | 5.18 | 3.65 |
| 44 | 4.57 | 4.06 |

**Example 19**

Pilot biodistribution and relaxometric studies of liposomal GdDTPA-BMA in male rats

a) Intramuscular injection

DPPC/DPPC liposomes containing GdDTPA-BMA (prepared in Example 18) were injected intramuscularly (im) into Sprague Dawley rats at a dosage of 20 ÷μmol/kg.
Table 14 shows the $T_1$ relaxation times (37°C, 0.235 T) of excised tissues and blood one and three hours after im injection of DPPC/DPPG liposomes (n= 2x3). *Table 15* shows the temperature response of the $T_1$ in muscle. All results are given as mean values.

Table 14

| Time post Injection (min) | $T_1$ relaxation time (ms) | | |
|---|---|---|---|
| 0 | 500 | 833 | 248 |
| 60 | 451 | 950 | 249 |
| 180 | 440 | 833 | 243 |

Table 15

| Time post Injection (min) | Muscle $T_1$ (ms) | |
|---|---|---|
| 0 | 500 | 340 |
| 60 | 451 | 341 |
| 180 | 440 | 383 |

Despite large interindividual variations, the results show the temperature dependence of the muscle $T_1$ after im administration of liposome encapsulated GdDTPA-BMA.

b) Intravenous injection

DPPC/DPPG/DPPE-PEG-2000 and DPPC/DPPG liposomes containing GdDTPA-BMA (prepared in *Example 18*) were injected intravenously (iv) into Sprague Dawley rats at a dosage of 100 μmol/kg.
Tables 16 and 17 show the $T_1$ relaxation times (37°C, 0.235 T) of excised tissues and blood, 5 minutes (n=3), one (n=2) and three (n=3) hours after iv injection of DPPC/DPPG and DPPC/DPPG/DPPE-PEG-2000 liposomes, respectively. Also shown, is the Gd uptake in tissue, expressed as the percentage tissue uptake of the administered Gd dosage. Tables 18 and 19 summarize the temperature response of the blood $T_1$ after iv injection of DPPC/DPPG and DPPC/DPPG/DPPE-PEG-2000 liposomes, respectively. A more detailed investigation of the temperature response was performed in blood withdrawn one hour after iv administration (n=3) of DPPC/DPPG liposomes, as shown in Table 20. All results are given as mean values.

Table 16

| Time post injection (min) | $T_1$ relaxation time (ms) Tissue uptake (% of adm. Gd dosage) | | | |
|---|---|---|---|---|
| | Liver | Blood | Spleen | Lungs |
| 0 | 248 | 833 | 510 | 613 |
| | - | - | - | - |
| 5 | 212 | 533 | 357 | 547 |
| | 11.6 | 60.0 | 1.9 | 0.88 |
| 60 | 223 | 589 | 315 | 520 |
| | 13.7 | 25.9 | 4.3 | 0.48 |
| 180 | 227 | 823 | 353 | 557 |
| | 7.9 | 1.3 | 2.0 | 0.06 |

Table 17

| Time post injection (min) | $T_1$ relaxation time (ms) Tissue uptake (% of adm. Gd dosage) | | | |
|---|---|---|---|---|
| | Liver | Blood | Spleen | Lungs |
| 0 | 248 | 833 | 510 | 613 |
| | - | - | - | - |
| 5 | 224 | 540 | 417 | 580 |
| | 5.7 | 64.3 | 1.4 | 0.85 |
| 180 | 202 | 500 | 298 | 563 |
| | 8.8 | 27.7 | 6.5 | 0.56 |

Table 18

| Time post Injection (min) | Blood $T_1$ (ms) | |
|---|---|---|
| | 37°C | 43°C |
| 0 | 833 | 880 |
| 5 | 533 | 151 |
| 60 | 589 | 273 |
| 180 | 823 | 797 |

Table 19

| Time post Injection (min) | Blood $T_1$ (ms) | |
|---|---|---|
| | 37°C | 43°C |
| 0 | 833 | 880 |
| 60 | 540 | 135 |
| 180 | 500 | 244 |

Table 20

| Blood $T_1$ (ms) | | | |
|---|---|---|---|
| 37°C | 40°C | 41°C | 43°C |
| 520 | 247 | 232 | 210 |

The results show the potential of both non-PEGylated and, especially, PEGylated liposomal GdDTPA-BMA as blood pool agents. The $T_1$-temperature sensitivity of the liposomes was also demonstrated in blood.

**Example 20**

*In Vitro* Imaging Studies with GdDTPA-BMA encapsulated within DSPC/DPPC/DPPG liposomes

DSPC/DPPC/DPPG liposomes containing GdDTPA-BMA were prepared analogously to Example 2. The liposome size (z-average) was 129 nm. MR imaging was performed at 2.0 T (Bruker Medspec) on a concentric spherical phantom in which the inner chamber contained liposomal GdDTPA-BMA diluted with an isotonic medium composed of glucose and 6.25% polyvinylpyrrolidone (conc. ≈0.8 mM Gd), whilst the outer compartment was filled with saline. Microwave heating was performed at 434 MHz with a linear radio frequency antenna placed in the outer chamber. The microwave irradiation was applied simultaneously with the image acquisition. Blocks consisting of 10 diffusion-weighted spin-echo single shot EPI (DW-SE-EPI) images (b-factor from 3 to 864 s/mm$^2$), a set of SE-EPI images with inversion-recovery preparation (IR-SE-EPI) and gradient echo $T_1$-weighted ($T_1$W-GE) images were repeated until the temperature of the liposome sample reached 48°C (in appr. 110 min). $T_1$W-GE images were acquired with TE/TR/flip: 5ms/30ms/50°. $T_1$-maps were calculated from the set of 13 IR-SE-EPI images, measured with inversion times varying from 14.4 ms to 16 s. Plots of $1/T_1$ ($R_1$) versus temperature were generated from a fixed region-of-interest within the phantom. The sample temperature was measured by a thermocouple immediately after acquisition of each block. The temperature distribution within the imaged slice was evaluated from ADC-maps.

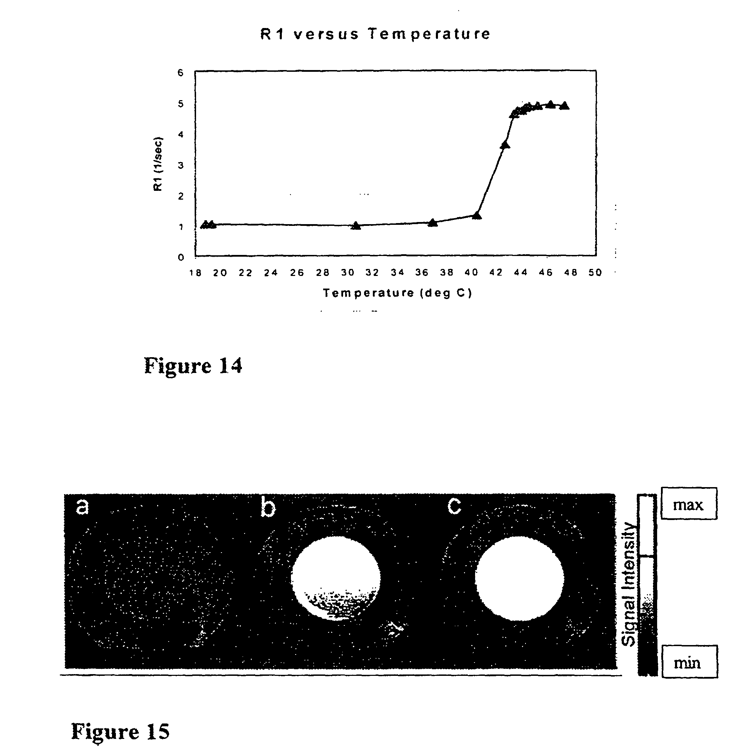

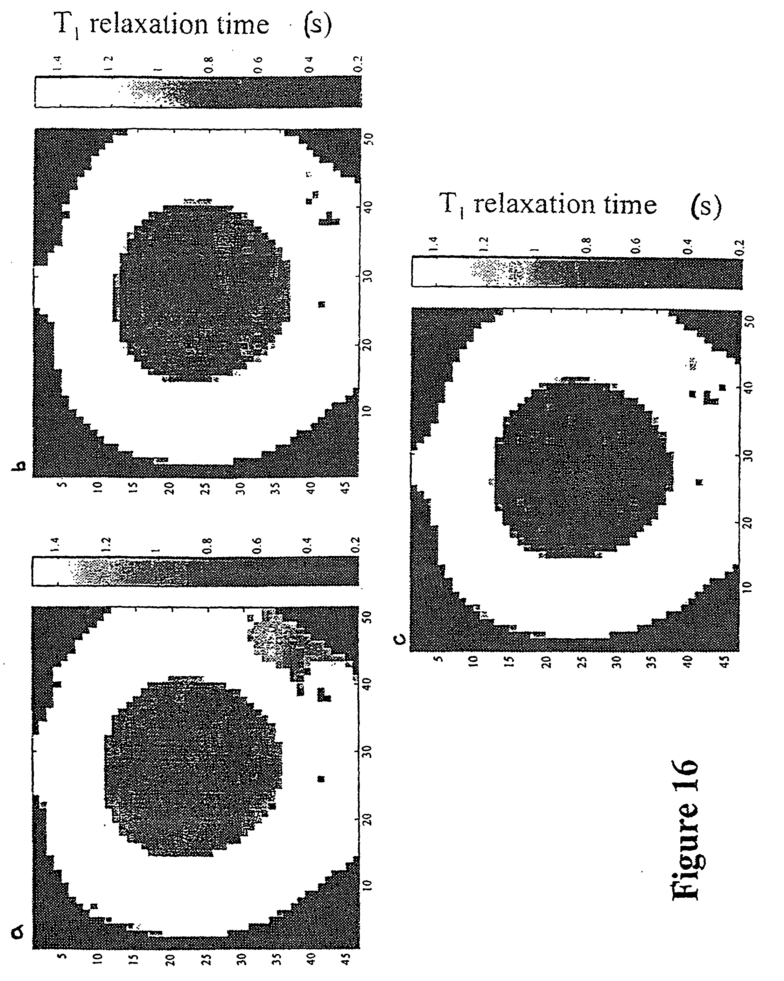

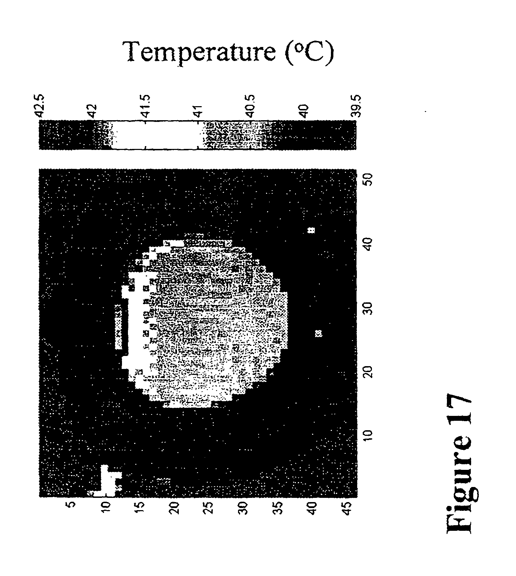

The temperature evolution of the measured $R_1$ for liposomal GdDTPA-BMA is summarized in Figure 14. A linear correlation was obtained between $R_1$ and temperature in the "transition region" 40.4-43.7°C (regression coefficient of 0.995). Figure 15 shows selected $T_1$-W GE images of the phantom (a) before heating, (b) during heating; signal intensity distribution observed within liposome sample, and (c) after heating; homogeneous signal intensity distribution. Figure 16 shows the corresponding $T_1$-maps at the same time points as for Figure 15. By use of the linear correlation between $R_1$ and temperature, a corresponding temperature map could be derived from the $T_1$-map at timepoint (b), as seen on Figure 17. The temperature map demonstrates the thermosensitivity of liposomal GdDTPA-BMA. (NB The temperature scale is only valid for the inner chamber containing liposomes).

**Example 21**

In vitro MR imaging studies with GdDTPA-BMA encapsulated within DPPC/DPPG liposomes - Determination of Gd concentration.

A static *in vitro* phantom, composed of twelve glass vials (10 mm dia.) placed in a rectangular plastic container, was used for this study. The plastic container was filled with a viscous isotonic medium composed of glucose/25% (w/w) polyvinyl-pyrrolidone (PVP) and doped with GdDTPA-BMA to give a $T_1$ of about 430 ms at 1.5 T. Three of the vials contained a marker solution with a known $T_1$ value (about 630 ms). The remaining nine vials were filled with DPPC/DPPG-based GdDTPA-BMA liposomes (prepared in Example 18) dispersed in varying amounts of isotonic 10% PVP/glucose solution. The Gd concentration [Gd] in the liposome samples ranged from 0 to 5.2 mM Gd as determined by inductively coupled plasma atomic emission spectrophotometry. The phantom was imaged at room temperature in a quadrature knee coil at 1.5 T on a Philips NT system. The following imaging sequences were used:

1. $T_1$-FFE (spoiled gradient echo) TR/TE/flip: 15ms/2ms/30°.
2. TMIX (quantitative $T_1/T_2$ sequence)
3. Dual TE FFE ($T_2$* mapping) (TE1/TE2: 4ms/50 ms)

All three sequences were repeated after heating of the phantom, the latter achieved by placing the phantom in a warm (>60°C) water bath. The temporal effect of heating was not investigated, only the end-effect (i.e. T >> $T_c$).
A linear correlation was obtained between [Gd] and matrix corrected $R_1$ ($\Delta R_1$) prior to heating (measured from TMIX sequence), with a regression coefficient of 0.996. The calculated liposomal $r_1$ was 0.11 $mM^{-1}s^{-1}$. Analogously, a liposomal $r_2$ of 0.55 $mM^{-1}s^{-1}$ was determined from the $R_2$ vs [Gd] curve (regression coefficient = 0.997); the $r_2/r_1$ ratio being equal to 5. After liposome heating, the $r_1$ and $r_2$ were 3.23 and 3.75, respectively, giving an $r_2/r_1$ ratio of 1.16.

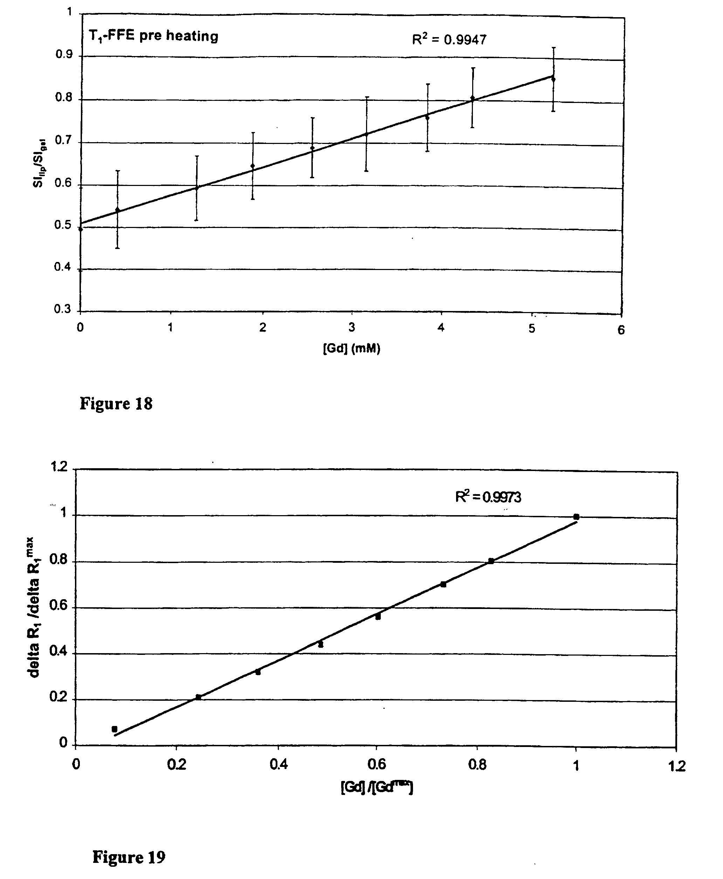

Figure 18 shows, prior to heating, a linear correlation between [Gd] and the ratio of the signal intensities of liposome sample and PVP gel ($SI_{lip}/SI_{gel}$) using the $T_1$-FFE sequence. Figure 19 shows the plot of the $\Delta R_1/\Delta R_1^{max}$ ratio vs the [Gd]/[Gd$^{max}$] ratio; here [Gd$^{max}$] = 5.2 mM. The results demonstrated that prior to heating, the $\Delta R_1$ ratio accurately reflected the [Gd] ratio. Similar results were also obtained when the $\Delta R_2$ ratio was employed. The findings suggest that the $T_1$- (and $T_2$-) effect of liposome encapsulated GdDTPA-BMA prior to heating is significant enough to enable a relative assessment of liposomal Gd concentration.

**Example 22**

*In vitro* MR imaging studies with GdDTPA-BMA encapsulated within DPPE/PA liposomes

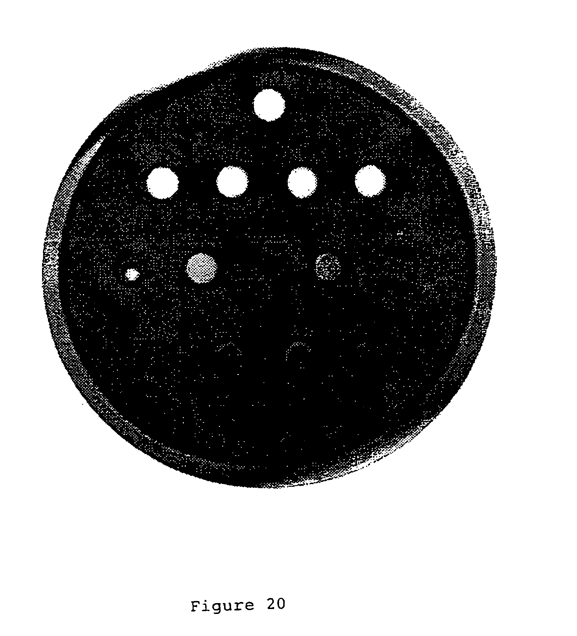

DPPE/PA liposomes containing GdDTPA-BMA were prepared analogously to Example 9. The mean hydrodynamic diameter of the liposomes was measured to 158 nm by photon correlation spectroscopy (Malvern PS/MW 4700, Malvern Instruments Ltd., Malvern, England). An *in vitro* phantom, composed of thirteen glass vials (11 mm diameter) placed in a circular glass reactor, was used for this study. The glass reactor was filled with an agar gel (2 % w/v) doped with GdDTPA-BMA to give a $T_1$ of about 900 ms at 1.5 T. The glass vials were filled with isoosmotic buffer solutions with pHs ranging from 4.8 to 8.2. The phantom was constantly held at a temperature of 37°C by circulating heated water through the shell of the reactor with a circulating water pump. Liposomes were added successively to each vial with a time interval of 1 minute. The imaging was started 25 minutes after addition of liposomes to the first vial. The phantom was imaged at 1.5 T on a Philips NT system. The following imaging parameters were used: sequence: MIX-TSE; TR (ms): 800.0; TE (ms): 12.5; TI (ms): 500.0; flip (deg): 90; slice thickness (mm): 7.0; FoV (freq*phase, mm): 230.0*230.0. The scan cycles were repeated every minute for 20 minutes. Figure 20 shows the phantom 25 minutes after addition of liposomes to the first vial. The signal intensity increases with decreasing pH.

**Example 26**

GdDTPA-BMA encapsulated within $Mg^{2+}$ sensitive liposomes

Beef-heart cardiolipin - cesium salt, dipalmitoylphosphatidylcholine (DPPC) and dipalmitoylphosphatidylglycerol - potassium salt (DPPG) are added to a round bottom flask in 40/55/5 mol ratio (totally 500 mg) and dissolved using chloroform. The chloroform is removed by evaporation under reduced pressure using a rotavapor. Liposomes are formed by hydrating the lipid film with a pre-heated(52°C) aqueous solution of 250 mM GdDTPA-BMA (10 ml). The liposomes are subjected to 3 freeze-thaw cycles and allowed to swell for one and half-hour at 55°C. The liposome dispersion is extruded at 62°C through polycarbonate filters of various pore diameters. Untrapped GdDTPA-BMA is removed by dialysis against isoosmotic and isoprotic glucose solution

The liposome dispersion is diluted ten times with water and transformed to an NMR tube. The $r_1$ relaxivity at 0.235 Tesla is measured using a Minispec NMR instrument at 37°C. The r1 relaxivitiy is low. The sample is then titrated by a 0.6 M $MgCl_2$ solution. When the $Mg^{2+}$ to cardiolipin ratio increases, the lamellar to $H_{II}$ phase transition is induced as described in F. Reiss-Husson, J. Mol. Biol., **25**, 363, (1967). The break-down of the liposomal structure leads to contact between the GdDTPA-BMA and water, inducing a significant increase in the r1 relaxtivitiy. This experiment will demonstrate a $Mg^{2+}$ sensitive MRI contrast agent.

**Example 27**

GdDTPA-BMA encapsulated within $Ca^{2+}$ sensitive liposomes

The experiment as described in Example 26 above is repeated, but the $MgCl_2$ solution is replaced by a $CaCl_2$ solution. Observations of a similar increase in relaxivity at a sufficiently high $Ca^{2+}$ concentration demonstrate a $Ca^{2+}$ sensitive MRI contrast agent.

**Example 30**

Gadolinium DTPA labelled starch microspheres

Gadolinium DTPA starch particles were prepared according to P. Rongved et al. in Carbohydrate Research 214 (1991) 325-330 substrate 9 to 12. The particles were suspended in 0.9 % NaCl solution before administration. The product can be used to diagnose diseases related to abnormal enzyme activity ($\alpha$-amylase and esterase); for example.

**Claims**

1. A contrast medium for imaging of a physiological parameter, said medium comprising a particulate material comprising a matrix or membrane material and at least one magnetic resonance contrast generating species, said matrix or membrane material being responsive to a pre-selected physiological parameter and the response is an increased matrix or membrane permeability or chemical or physical breakdown of the matrix or membrane material, to cause the contrast efficacy of said contrast generating species to vary in response to said parameter.

2. A contrast medium as claimed in claim 1 wherein the matrix or membrane material is responsive to pH, temperature, pressure, carbon dioxide tension, oxygen tension, enzyme activity, tissue electrical activity, tissue water diffusion or ion concentration.

3. A contrast medium as claimed in claim 2 wherein the matrix or membrane material is responsive to pH, temperature or pressure.

4. A contrast medium as claimed in claim 1 to 3 wherein the magnetic resonance contrast generating species is selected from the group consisting of paramagnetic compounds, superparamagnetic compounds, ferrimagnetic compounds, ferromagnetic compounds and compounds containing other non-zero spin nuclei than hydrogen.

5. A contrast medium as claimed in claim 4 wherein the magnetic resonance contrast generating species is a paramagnetic compound.

6. A contrast medium as claimed in claim 5 wherein the magnetic resonance contrast generating species is a paramagnetic compound selected from the group consisting of stable free radicals, transition metal compounds and lanthanide metal compounds.

7. A contrast medium as claimed in claim 5 wherein the magnetic resonance contrast generating species is a paramagnetic compound selected from the group consisting of manganese compounds, gadolinium chelates, ytterbium chelates, dysprosium chelates and europium compounds.

8. A contrast medium as claimed in claim 4 wherein the magnetic resonance contrast generating species is a superparamagnetic metal oxide.

9. A contrast medium as claimed in claim 4 wherein the magnetic resonance contrast generating species is a compound containing other non-zero spin nuclei than hydrogen selected from the group consisting of $^{19}$F, $^{13}$C, $^{15}$N, $^{29}$Si and $^{31}$P.

10. A contrast medium as claimed in claim 9 wherein the magnetic resonance contrast generating species is a compound containing $^{19}$F.

11. A contrast medium as claimed in claim 9 wherein the magnetic resonance contrast generating species is a compound containing $^{13}$C or $^{15}$N.

12. A contrast medium as claimed in claim 9 to 11 wherein the non-zero spin nuclei are hyperpolarized nuclei.

13. A contrast medium as claimed in claim 1 to 12 wherein the matrix or membrane material is selected from lipids, phospholipids, surfactants, proteins, oligomers or physiologically acceptable polymers.

**14.** A contrast medium as claimed in claim 13 wherein the matrix or membrane material forms a vesicle or a liposome.

**15.** A contrast medium as claimed in claim 13 wherein the matrix or membrane material is responsive to pH.

**16.** A contrast medium as claimed in claim 13 wherein the matrix or membrane material is responsive to temperature.

**17.** A contrast medium as claimed in claim 16 wherein the matrix or membrane material comprises a lipid or a lipid mixture or a phospholipid or a phospholipid mixture.

**18.** A contrast medium as claimed in claim 17 wherein the lipid or the lipid mixture or the phospholipid or the phospholipid mixture has a Tc value between 35°C and 80°C.

**19.** A contrast medium as claimed in claims 1 to 19 wherein said particulate material is in combination with a targeting ligand for a cell or receptor of interest.

**20.** A contrast medium as claimed in claim 1 wherein the matrix or membrane material is responsive to temperature and comprises hydrogenated phosphatidyl choline (HPC), hydrogenated phosphatidylserine-sodium (HPS), dipalmitoylphophatidylcholine (DPPC), distearylphosphatidylcholine (DSPC), dipalmitoylphosphatidylglycerol (DPPG), dipalmitoylphosphatidylethanolamine (DPPE), dibehenoylphosphatidylcholine, dimyristoylphosphatidyl glycerol (DMPG), cholesterol, cardiolipin and starch and the magnetic resonance contrast generating species is selected from the group consisting of superparamagnetic iron oxide, GdDTPA-BMA GdBOPTA, GdDTPA, GdDOTA, GdHPD03A, DyDTPA-BMA and PrD03A.

**21.** Use of a particulate material comprising a matrix or membrane material and at least one magnetic resonance contrast generating species, said matrix or membrane material being responsive to temperature and the response is an increased matrix or membrane permeability or chemical or physical breakdown of the matrix or membrane material for the manufacture of a contrast medium for use in a method of diagnosing cancer, cardiovascular diseases or inflammation comprising generating a signal indicative of the value of temperature.

**22.** Use of a particulate material comprising a matrix or membrane material and at least one magnetic resonance contrast generating species, said matrix or membrane material being responsive to pH and the response is an increased matrix or membrane permeability or chemical or physical breakdown of the matrix or membrane material for the manufacture of a contrast medium for use in a method of diagnosing cancer, cardiovascular diseases, osteoporosis, inflammation or autoimmune diseases comprising generating a signal indicative of the value of pH.

**23.** Use of a particulate material comprising a matrix or membrane material and at least one magnetic resonance contrast generating species, said matrix or membrane material being responsive to temperature and the response is an increased matrix or membrane permeability or chemical or physical breakdown of the matrix or membrane material for the manufacture of a contrast medium for use in a method of monitoring hyperthermia treatment.

**24.** Use according to claim 23 wherein the hyperthermia treatment is carried out by external heating, preferably by using focused ultrasound.

**Patentansprüche**

**1.** Kontrastmedium zum Abbilden eines physiologischen Parameters, wobei das Medium ein teilchenförmiges Material umfasst, welches ein Matrix- oder Membranmaterial und mindestens eine Spezies, welche einen Magnetresonanzkontrast bildet, umfasst, wobei das Matrix- oder Membranmaterial auf einen vorher ausgewählten physiologischen Parameter anspricht und die Antwort eine erhöhte Matrix- oder Membranpermeabilität oder ein chemischer oder physikalischer Zusammenbruch des Matrix- oder Membranmaterials ist, wodurch eine Variation der Kontrastwirksamkeit der kontrastbildenden Spezies in der Antwort auf den Parameter hervorgerufen wird.

**2.** Kontrastmedium nach Anspruch 1, worin das Matrix- oder Membranmaterial auf den pH, Temperatur, Druck, Kohlendioxidspannung, Sauerstoffspannung, Enzymaktivität, Gewebeelektroaktivität, Gewebewasserdiffusion oder Ionenkonzentration anspricht.

**3.** Kontrastmedium nach Anspruch 2, worin das Matrix- oder Membranmaterial auf den pH, Temperatur oder Druck anspricht.

4.  Kontrastmedium nach Anspruch 1 bis 3, worin die Spezies, welche einen Magnetresonanzkontrast bildet, ausgewählt ist aus der Gruppe, bestehend aus paramagnetischen Verbindungen, superparamagnetischen Verbindungen, ferrimagnetischen Verbindungen, ferromagnetischen Verbindungen und Verbindungen, welche andere Nicht-Null-Spin-Kerne als Wasserstoff enthalten.

5.  Kontrastmedium nach Anspruch 4, worin die Spezies, welche einen Magnetresonanzkontrast bildet, eine paramagnetische Verbindung ist.

6.  Kontrastmedium nach Anspruch 5, worin die Spezies, welche einen Magnetresonzanzkontrast bildet, eine paramagnetische Verbindung, ausgewählt aus der Gruppe, bestehend aus stabilen freien Radikalen, Übergangsmetallverbindungen und Lanthanidmetallverbindungen, ist.

7.  Kontrastmedium nach Anspruch 5, worin die Spezies, welche einen Magnetresonanzkontrast bildet, eine paramagnetische Verbindung, ausgewählt aus der Gruppe, bestehend aus Manganverbindungen, Gadoliniumchelaten, Ytterbiumchelaten, Dysprosiumchelaten und Europiumverbindungen, ist.

8.  Kontrastmedium nach Anspruch 4, worin die Spezies, welche einen Magnetresonanzkontrast bildet, ein superparamagnetisches Metalloxid ist.

9.  Kontrastmedium nach Anspruch 4, worin die Spezies, welche einen Magnetresonanzkontrast bildet, eine Verbindung, welche andere Nicht-Null-Spin-Kerne als Wasserstoff enthält, ausgewählt aus der Gruppe, bestehend aus $^{19}$F, $^{13}$C, $^{15}$N, $^{29}$Si und $^{31}$P, ist.

10. Kontrastmedium nach Anspruch 9, worin die Spezies, welche einen Magnetresonanzkontrast bildet, eine Verbindung, welche $^{19}$F enthält, darstellt.

11. Kontrastmedium nach Anspruch 9, worin die Spezies, welche einen Magnetresonanzkontrast bildet, eine Verbindung, welche $^{13}$C oder $^{15}$N enthält, darstellt.

12. Kontrastmedium nach einem der Ansprüche 9 bis 11, worin die Nicht-Null-Spin-Kerne hyperpolarisierte Kerne sind.

13. Kontrastmedium nach einem der Ansprüche 1 bis 12, worin das Matrix- oder Membranmaterial ausgewählt ist aus Lipiden, Phospholipiden, Tensiden, Proteinen, Oligomeren oder physiologisch annehmbaren Polymeren.

14. Kontrastmedium nach Anspruch 13, worin das Matrix- oder Membranmaterial ein Vesikel oder ein Liposom bildet.

15. Kontrastmedium nach Anspruch 13, worin das Matrix- oder Membranmaterial auf den pH anspricht.

16. Kontrastmedium nach Anspruch 13, worin das Matrix- oder Membranmaterial auf die Temperatur anspricht.

17. Kontrastmedium nach Anspruch 16, worin das Matrix- oder Membranmaterial ein Lipid oder eine Lipidmischung oder ein Phospholipid oder eine Phospholipidmischung umfasst.

18. Kontrastmedium nach Anspruch 17, worin das Lipid oder die Lipidmischung oder das Phospholipid oder die Phospholipidmischung einen Tc-Wert zwischen 35°C und 80°C aufweist.

19. Kontrastmedium nach einem der Ansprüche 1 bis 19, worin das teilchenförmige Material in Kombination mit einem Zielliganden für eine Zelle oder einen Rezeptor von Interesse vorliegt.

20. Kontrastmedium nach Anspruch 1, worin das Matrix- oder Membranmaterial gegenüber der Temperatur anspricht und hydriertes Phosphatidylcholin (HPC), hydriertes Phosphatidylserin-Natrium (HPS), Dipalmitoylphosphatidylcholin (DPPC), Distearylphosphatidylcholin (DSPC), Dipalmitoylphosphatidylglycerin (DPPG), Dipalmitoylphosphatidylethanolamin (DPPE), Dibehenoylphosphatidylcholin, Dimyristoylphosphatidylglycerin (DMPG), Cholesterin, Cardiolipin und Stärke umfasst und worin die Spezies, welche einen Magnetresonanzkontrast bildet, ausgewählt ist aus der Gruppe, bestehend aus superparamagnetischem Eisenoxid, GdDTPA-BMA, GdBOPTA, GdDTPA, GdDOTA, GdHPDO3A, DyDTPA-BMA und PrD03A.

**21.** Verwendung eines teilchenförmigen Materials, welches ein Matrix- oder Membranmaterial und mindestens eine Spezies, welche einen Magnetresonanzkontrast bildet, umfasst, wobei das Matrix- oder Membranmaterial gegenüber der Temperatur anspricht und die Antwort eine erhöhte Matrix- oder Membranper- meabilität oder ein chemischer oder physikalischer Zusammenbruch des Matrixoder Membranmaterials ist, zur Herstellung eines Kontrastmediums zur Verwendung in einem Verfahren zur Diagnose von Krebs, kardiovaskulärer Erkrankungen oder Entzündungen, umfassend die Bildung eines Signals, welches den Wert der Temperatur anzeigt.

**22.** Verwendung eines teilchenförmigen Materials, welches ein Matrix- oder Membranmaterial und mindestens eine Spezies, welche einen Magnetresonanzkontrast bildet, umfasst, wobei das Matrix- oder Membranmaterial gegenüber dem pH anspricht und die Antwort eine erhöhte Matrix- oder Membranpermeabilität oder ein chemischer oder physikalischer Zusammenbruch des Matrix- oder Membranmaterials ist, zur Herstellung eines Kontrastmediums zur Verwendung in einem Verfahren zur Diagnose von Krebs, kardiovaskulärer Erkrankungen, Osteoporose, Entzündungen oder Autoimmun-Erkrankungen, umfassend die Bildung eines Signals, welches den Wert des pH angibt.

**23.** Verwendung eines teilchenförmigen Materials, welches ein Matrix- oder Membranmaterial und mindestens eine Spezies, welche einen Magnetresonanzkontrast bildet, umfasst, wobei das Matrix- oder Membranmaterial gegenüber der Temperatur anspricht und die Antwort eine erhöhte Matrix- oder Membranpermeabilität oder ein chemischer oder physikalischer Zusammenbruch des Matrix- oder Membranmaterials ist, zur Herstellung eines Kontrastmediums zur Verwendung in einem Verfahren zur Hyperthermie-Behandlung.

**24.** Verwendung nach Anspruch 23, worin die Hyperthermie-Behandlung durch externes Erwärmen, vorzugsweise unter Verwendung von fokussiertem Ultraschall, durchgeführt wird.

## Revendications

**1.** Milieu de contraste pour imager un paramètre physiologique, ledit milieu comprenant une matière particulaire comprenant une matière de matrice ou de membrane et au moins une espèce générant un contraste par résonance magnétique, ladite matière de matrice ou de membrane réagissant à un paramètre physiologique prédéterminé et la réponse est une perméabilité accrue de la matrice ou de la membrane ou une décomposition chimique ou physique de la matière de matrice ou de membrane, pour faire en sorte que l'efficacité du contraste de ladite espèce générant un contraste varie en réponse audit paramètre.

**2.** Milieu de contraste selon la revendication 1, dans lequel la matière de matrice ou de membrane réagit au pH, à la température, à la pression, à la tension en dioxyde de carbone, à la tension en oxygène, à l'activité enzymatique, à l'activité électrique tissulaire, à la diffusion tissulaire de l'eau ou à la concentration ionique.

**3.** Milieu de contraste selon la revendication 2, dans lequel la matière de matrice ou de membrane réagit au pH, à la température ou à la pression.

**4.** Milieu de contraste selon les revendications 1 à 3, dans lequel l'espèce générant un contraste par résonance magnétique est choisie dans le groupe constitué par les composés paramagnétiques, les composés superparamagnétiques, les composés ferrimagnétiques, les composés ferromagnétiques et les composés contenant des noyaux à spin non nul autres que l'hydrogène.

**5.** Milieu de contraste selon la revendication 4, dans lequel l'espèce générant un contraste par résonance magnétique est un composé paramagnétique.

**6.** Milieu de contraste selon la revendication 5, dans lequel l'espèce générant un contraste par résonance magnétique est un composé paramagnétique choisi dans le groupe constitué par les radicaux libres stables, des composés de métaux de transition et des composés des métaux des lanthanides.

**7.** Milieu de contraste selon la revendication 5, dans lequel l'espèce générant un contraste par résonance magnétique est un composé paramagnétique choisi dans le groupe constitué par les composés du manganèse, les chélates de gadolinium, les chélates d'ytterbium, les chélates de dysprosium et les composés d'europium.

**8.** Milieu de contraste selon la revendication 4, dans lequel l'espèce générant un contraste par résonance magnétique est un oxyde métallique superparamagnétique.

9. Milieu de contraste selon la revendication 4, dans lequel l'espèce générant un contraste par résonance magnétique est un composé contenant des noyaux à spin non nul autres que l'hydrogène choisis dans le groupe constitué par $^{19}F$, $^{13}C$, $^{15}N$, $^{29}Si$ et $^{31}P$.

10. Milieu de contraste selon la revendication 9, dans lequel l'espèce générant un contraste par résonance magnétique est un composé contenant $^{19}F$.

11. Milieu de contraste selon la revendication 9, dans lequel l'espèce générant un contraste par résonance magnétique est un composé contenant $^{13}C$ ou $^{15}N$.

12. Milieu de contraste selon les revendications 9 à 11, dans lequel les noyaux à spin non nul sont des noyaux hyperpolarisés.

13. Milieu de contraste selon les revendications 1 à 12, dans lequel la matière de matrice ou de membrane est choisie parmi les lipides, les phospholipides, les agents tensioactifs, les protéines, les oligomères, ou les polymères physiologiquement acceptables.

14. Milieu de contraste selon la revendication 13, dans lequel la matière de matrice ou de membrane forme une vésicule ou un liposome.

15. Milieu de contraste selon la revendication 13, dans lequel la matière de matrice ou de membrane réagit au pH.

16. Milieu de contraste selon la revendication 13, dans lequel la matière de matrice ou de membrane réagit à la température.

17. Milieu de contraste selon la revendication 16, dans lequel la matière de matrice ou de membrane comprend un lipide ou un mélange de lipides ou un phospholipide ou un mélange de phospholipides.

18. Milieu de contraste selon la revendication 17, dans lequel le lipide ou le mélange de lipides ou le phospholipide ou le mélange de phospholipides a une valeur Tc entre 35 °C et 80 °C.

19. Milieu de contraste selon les revendications 1 à 19, dans lequel ladite matière particulaire est combinée avec un ligand ciblant pour une cellule ou un récepteur d'intérêt.

20. Milieu de contraste selon la revendication 1, dans lequel la matière de matrice ou de membrane réagit à la température et comprend de la phosphatidylcholine hydrogénée (HPC), de la phosphatidylsérine sodique hydrogénée (HPS), de la dipalmitoylphosphatidyl- choline (DPPC), de la distéarylphosphatidylcholine (DSPC), du dipalmitoylphosphatidylglycérol (DPPG), de la dipalmitoylphosphatidyléthanolamine (DPPE), de la dibéhénoylphosphatidylcholine, du dimyristoylphosphatidylglycérol (DMPG), du cholestérol, de la cardiolipine et de l'amidon, et l'espèce générant un contraste par résonance magnétique est choisie dans le groupe constitué par l'oxyde de fer superparamagnétique, GdDTPA-BMA, GdBOPTA, GdDTPA, GdDOTA, GdHPD03A, DyDTPA-BMA et PrD03A.

21. Utilisation d'une matière particulaire comprenant une matière de matrice ou de membrane et au moins une espèce générant un contraste par résonance magnétique, ladite matière de matrice ou de membrane réagissant à la température et la réponse étant une perméabilité accrue de la matrice ou de la membrane ou une décomposition chimique ou physique de la matière de matrice ou de membrane, pour la fabrication d'un milieu de contraste pour l'utilisation dans un procédé de diagnostic du cancer, de maladies cardiovasculaires ou d'une inflammation comprenant la production d'un signal indiquant la valeur de température.

22. Utilisation d'une matière particulaire comprenant une matière de matrice ou de membrane et au moins une espèce générant un contraste par résonance magnétique, ladite matière de matrice ou de membrane réagissant au pH et la réponse est une perméabilité accrue de la matrice ou de la membrane ou une décomposition chimique ou physique de la matière de matrice ou de membrane, pour la fabrication d'un milieu de contraste pour l'utilisation dans un procédé de diagnostic du cancer, de maladies cardiovasculaires, de l'ostéroporose, d'une inflammation ou de maladies autoimmunes comprenant la production d'un signal indiquant la valeur de pH.

**23.** Utilisation d'une matière particulaire comprenant une matière de matrice ou de membrane et au moins une espèce générant un contraste par résonance magnétique, ladite matière de matrice ou de membrane réagissant à la température et la réponse est une perméabilité accrue de la matrice ou de la membrane ou une décomposition chimique ou physique de la matière de matrice ou de membrane, pour la fabrication d'un milieu de contraste pour l'utilisation dans un procédé de suivi d'un traitement de l'hyperthermie.

**24.** Utilisation selon la revendication 23, dans laquelle le traitement de l'hyperthermie est mis en oeuvre par chauffage externe, de préférence en utilisant un ultrason focalisé.

FIG. 1

TEMPERATURE RESPONSE OF <u>IN VITRO</u> $r_1$ FOR GdDTPA-BMA
ENCAPSULATED IN DPPC/DPPG LIPOSOMES (0.47T)

EP 1 069 888 B1

## FIG. 2

TEMPERATURE RESPONSE OF MR SIGNAL INTENSITY FOR GdDTPA-BMA
ENCAPSULATED WITHIN DPPC/DPPG LIPOSOMES (2.0 T).

**Figure 3.** Gel phantom (a) containing inserts of DPPC\DPPG-based GdDTPA-BMA liposomes (labelled 3-10) and control glucose 5% solution (labelled 1-2); $T_1$-w GRE images (2.0 T) of phantom prior to (b), after (c) 47 and (d) 63 minutes of radiofrequency heating, inhomogeneous signal intensity in gel is due to air bubbles; (e) difference image after subtraction of (b) from (d). Note that the signal intensity from inserts 3 and 5 is almost unchanged after heating as the temperature never exceeded $T_c$.

EP 1 069 888 B1

FIG.4

FIG.5

FIG.6

FIG.7

FIG.8

FIG.9

FIG.10

FIG.11

FIG.12

FIG.13

## R1 versus Temperature

Figure 14

Figure 15

Figure 16

Figure 17

**Figure 18**

**Figure 19**

Figure 20